# EUROPÄISCHE PATENTSCHRIFT

(11) **EP 0 861 239 B1**
(45) Veröffentlichungstag und Bekanntmachung des Hinweises auf die Patenterteilung: **12.02.2003**
(21) Anmeldenummer: 96938113.6
(22) Anmeldetag: 06.11.1996
(51) Int. Cl.: C07D 263/14, A01N 43/76, C07D 413/12

(54) **BIPHENYLETHER-OXAZOLINE UND IHRE VERWENDUNG ALS SCHÄDLINGSBEKÄMPFUNGSMITTEL**
BIPHENYL ETHER OXAZOLINES AND THEIR USE AS PEST-CONTROL AGENTS
BIPHENYLETHER-OXAZOLINES ET LEUR UTILISATION COMME PARASITICIDES

(30) Priorität: 17.11.1995 DE 19542934; 18.09.1996 DE 19638047
(43) Veröffentlichungstag der Anmeldung: 02.09.1998
(73) Patentinhaber: Bayer CropScience AG, 40789 Monheim (DE)
(72) Erfinder: KRÄMER, Wolfgang, D-51399 Burscheid (DE); KRAATZ, Udo, D-51375 Leverkusen (DE); ERDELEN, Christoph, D-42799 Leichlingen (DE); WACHENDORFF-NEUMANN, Ulrike, D-56566 Neuwied (DE); TURBERG, Andreas, D-40699 Erkrath (DE); MENCKE, Norbert, D-51381 Leverkusen (DE)
(86) Internationale Anmeldenummer: EP9604846
(87) Internationale Veröffentlichungsnummer: WO97019067

(56) Entgegenhaltungen:
- EP-A- 0 432 661
- EP-A- 0 639 572
- EP-A- 0 696 584
- WO-A-96/11190
- WO-A-96/22283

## Beschreibung

Die vorliegende Erfindung betrifft neue Biphenylether-oxazoline, Verfahren zu ihrer Herstellung und ihre Verwendung zur Bekämpfung von tierischen Schädlingen sowie neue Zwischenprodukte.

Es ist bereits bekannt, dass bestimmte substituierte Biphenyl-oxazoline, wie z.B. 2-(2,6-Difluorphenyl)-4-(4'-methoxybiphenyl-4-yl)-1,3-oxazolin, insektizid und akarizid wirksam sind (vgl. EP-A 0 432 661).

Die Wirkungshöhe und/oder Wirkungsdauer dieser bekannten Verbindungen ist jedoch, insbesondere gegen bestimmte Organismen oder bei niedrigen Anwendungskonzentrationen, nicht in allen Anwendungsgebieten völlig zufriedenstellend.

Es wurden neue Biphenylether-oxazoline der Formel (I) 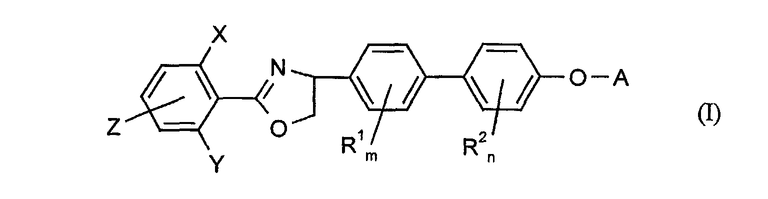 gefunden,
in welcher
- X: für Wasserstoff, Fluor oder Chlor steht,
- Y: für Fluor, Chlor oder Methyl steht,
- Z: für Wasserstoff, Fluor, Chlor, C₁-C₄-Alkyl, C₁-C₄-Alkoxy oder Di(C₁-C₄)alkylamino steht,
- R¹ und R²: unabhängig voneinander für Fluor, Chlor, C₁-C₄-Alkyl, C₁-C₄-Alkoxy, C₁-C₄-Alkylthio, C₁-C₄-Halogenalkyl, C₁-C₄-Halogenalkoxy oder C₁-C₄-Halogenalkylthio stehen,
- m und n: unabhängig voneinander für 0, 1 oder 2 stehen.
- A: steht für die Gruppierung (CH₂)ₚ-(CR³R⁴)_{q}-(CH₂)ᵣ-R,
wobei
R³ und R⁴ unabhängig voneinander für Wasserstoffoder C₁-C₄-Alkyl stehen,
p, q und r unabhängig voneinander für 0, 1, 2 oder 3 stehen, wobei mindestens ein Index ungleich 0 ist und die Summe der Indizes nicht größer als 5 ist,
- R: für Cyano; für einen der folgenden Heterocyclen 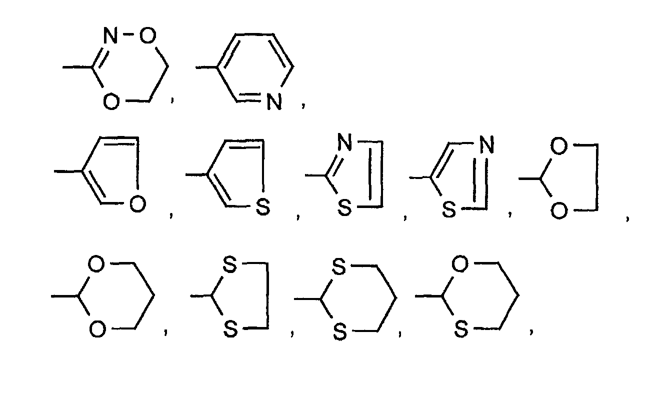 die gegebenenfalls einfach bis dreifach, gleich oder verschieden substituiert sind durch Fluor, Chlor, Brom, Methyl, Ethyl, n- oder i-Propyl oder Trifluormethyl steht;
oder für eine der folgenden Gruppierungen:
(a) ―CO-R⁵
(b) ―CO-OR⁶
(c) ―CO-NR⁷R⁸
(d) ―CS-NR⁷R⁸
(e) 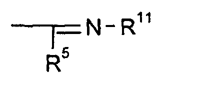
(f) 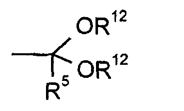
(g) 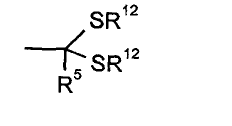
(h) 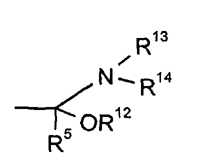
(i) 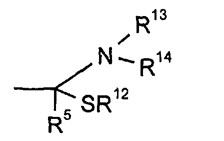
(j) 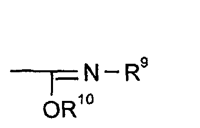
(k) 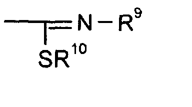
steht, wobei
R⁵ für Wasserstoff; Methyl, Ethyl, n- oder i-Propyl, die isomeren Butyle, die isomeren Pentyle, die isomeren Hexyle; C₁-C₂-Halogenalkyl mit 1 bis 5 gleichen oder verschiedenen Halogenatomen, wie F- und Cl-Atomen, C₃-C₆-Alkenyl, C₃-C₆-Halogenalkenyl mit 1 bis 5 gleichen oder verschiedenen Halogenatomen, wie F- und Cl-Atomen; jeweils gegebenenfalls einfach bis dreifach, gleich oder verschieden durch Fluor, Chlor, Methyl, Ethyl, n- oder i-Propyl oder C₁-C₂-Halogenalkyl mit 1 bis 5 gleichen oder verschiedenen Halogenatomen, wie F- und Cl-Atomen substituiertes Cyclopropyl, Cyclopentyl oder Cyclohexyl, oder für gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Methylcarbonylamino, Ethylcarbonylamino, Methylcarbonyl-methylamino, Cyano, Nitro, -COOR¹⁵ oder -CONR¹⁶R ¹⁷ substituiertes Phenyl steht,
R⁶ für Wasserstoff, Methyl, Ethyl, n- oder i-Propyl, n-, i-, s- oder t-Butyl; C₁-C₂-Halogenalkyl mit 1 bis 5 gleichen oder verschiedenen Halogenatomen, wie F- und Cl-Atomen; gegebenenfalls einfach oder mehrfach durch Fluor und/oder Chlor substituiertes Allyl; jeweils gegebenenfalls einfach bis dreifach, gleich oder verschieden durch Fluor, Chlor, Methyl, Ethyl, n- oder i-Propyl oder C₁-C₂-Halogenalkyl mit 1 bis 5 gleichen oder verschiedenen Halogenatomen, wie F- und Cl-Atomen substituiertes Cyclopropyl, Cyclopentyl, Cyclohexyl, Cyclopropyl-C₁-C₂-alkyl, Cyclopentyl-C₁-C₂-alkyl oder Cyclohexyl-C₁-C₂-alkyl,
oder für jeweils gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, -COOR¹⁵ oder -CONR ¹⁶R¹⁷ substituiertes Phenyl-C₁-C₂-alkyl oder Naphthyl-C₁-C₂-alkyl steht,
R⁷ und R⁸ unabhängig voneinander für Wasserstoff, C₁-C₄-Alkyl, Methoxy, Ethoxy, C₁-C₃-Halogenalkyl mit 1 bis 5 gleichen oder verschiedenen Halogenatomen, wie F- und Cl-Atomen; gegebenenfalls einfach oder mehrfach durch Fluor und/oder Chlor substituiertes Allyl; jeweils gegebenenfalls einfach bis dreifach, gleich oder verschieden durch Fluor, Chlor, Methyl, Ethyl, n- oder i-Propyl oder Trifluormethyl substituiertes Cyclopropyl, Cyclopentyl, Cyclohexyl, Cyclopropylmethyl, Cyclopentylmethyl oder Cyclohexylmethyl, oder für jeweils gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, -COOR¹⁵ oder -CONR ¹⁶R¹⁷ substituiertes Phenyl oder Phenyl-C₁-C₂-alkyl, oder für -OR⁶ oder -NR⁵R⁶ stehen,
R⁹ und R¹⁰ unabhängig voneinander für Methyl, Ethyl, n- oder i-Propyl stehen;
R¹¹ für -OR⁶, -NR⁵R⁶ oder -N(R⁵)-COOR⁶ steht, und
R¹², R¹³ und R¹⁴ unabhängig voneinander für Methyl, Ethyl, n- oder i-Propyl stehen,
- R¹⁵: für Wasserstoff, Methyl, Ethyl, n- oder i-Propyl; C₁-C₂-Halogenalkyl mit 1 bis 5 gleichen oder verschiedenen Halogenatomen, wie F- und Cl-Atomen; jeweils gegebenenfalls einfach bis dreifach, gleich oder verschieden durch Fluor, Chlor, Methyl, Ethyl, n- oder i-Propyl oder C₁-C₂-Halogenalkyl mit 1 bis 5 gleichen oder verschiedenen Halogenatomen, wie F- und Cl-Atomen substituiertes Cyclopropyl, Cyclopentyl oder Cyclohexyl, oder für gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, C₁-C₄-Alkoxycarbonyl oder Di(C₁-C₄)alkylaminocarbonyl substituiertes Phenyl steht,
- R¹⁶ und R¹⁷: unabhängig voneinander für Wasserstoff, C₁-C₄-Alkyl, Methoxy, Ethoxy, C₁-C₃-Halogenalkyl mit 1 bis 5 gleichen oder verschiedenen Halogenatomen, wie F- und Cl-Atomen; gegebenenfalls einfach oder mehrfach durch Fluor und/oder Chlor substituiertes Allyl; jeweils gegebenenfalls einfach bis dreifach, gleich oder verschieden durch Fluor, Chlor, Methyl, Ethyl, n- oder i-Propyl oder Trifluormethyl substituiertes Cyclopropyl, Cyclopentyl, Cyclohexyl, Cyclopropylmethyl, Cyclopentylmethyl oder Cyclohexylmethyl,
oder für jeweils gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, C₁-C₄-Alkoxycarbonyl oder Di(C₁-C₄)alkylaminocarbonyl substituiertes Phenyl oder Phenyl-C₁-C₂-alkyl
oder für -OR⁶ oder -NR⁵R⁶ stehen.

Die Biphenylether-oxazoline der Formel (I) können, auch in Abhängigkeit von den Substituenten, als optische und/oder geometrische Isomeren anfallen. Die vorliegende Erfindung betrifft sowohl die Isomerengemische als auch die reinen Isomeren.

Weiterhin wurde gefunden, dass man die Biphenylether-oxazoline der Formel (I) erhält, wenn man Hydroxibiphenyloxazoline der Formel (II) 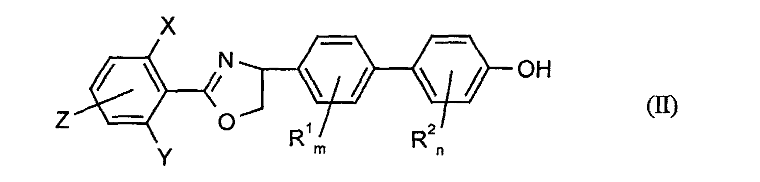 in welcher
- X, Y, Z, R¹, R², m und n: die oben angegebene Bedeutung haben,
mit einer Verbindung der Formel (III)

M-A (III)

in welcher
- A: die oben angegebene Bedeutung hat und
- M: für eine Abgangsgruppe steht,
gegebenenfalls in Gegenwart einer Base und/oder eines Katalysators sowie gegebenenfalls in Gegenwart eines Verdünnungsmittels umsetzt.

Weiter wurde gefunden, dass die neuen substituierten Biphenyletheroxazoline der Formel (I) sehr gut zur Bekämpfung von tierischen Schädlingen, insbesondere von Insekten, Spinnentieren und Nematoden, die in der Landwirtschaft, in Forsten, im Vorrats- und Materialschutz sowie auf dem Hygienesektor vorkommen, geeignet sind.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- X: steht besonders bevorzugt für Wasserstoff, Fluor oder Chlor.
- Y: steht besonders bevorzugt für Fluor oder Chlor.
- Z: steht besonders bevorzugt für Wasserstoff, Fluor, Chlor, Methyl, Ethyl, Methoxy, Dimethylamino oder Diethylamino.
- R¹ und R²: stehen unabhängig voneinander besonders bevorzugt für Fluor, Chlor, Methyl, Ethyl, Methoxy, Methylthio, Trifluormethyl, Trifluormethoxy oder Trifluormethylthio.
- m und n: stehen unabhängig voneinander besonders bevorzugt für 0, 1 oder 2.
- A: steht besonders bevorzugt für die Gruppierung (CH₂)ₚ-(CR³R⁴)_{q}-(CH₂)ᵣ-R,
wobei
R³ und R⁴ unabhängig voneinander für Wasserstoff, Methyl, Ethyl, n- oder i-Propyl, n-, i-, s- oder t-Butyl stehen;
p, q und r unabhängig voneinander für 0, 1, 2 oder 3 stehen, wobei mindestens ein Index ungleich 0 ist und die Summe der Indizes nicht größer als 5, insbesondere nicht größer als 3 ist.

Dabei gilt jeweils, dass für m = 2 und/oder n = 2 die Substituenten R¹ bzw. R² gleich oder verschieden sein können.

Die oben aufgeführten allgemeinen oder in Vorzugsbereichen aufgeführten Restedefinitionen bzw. Erläuterungen gelten für die Endprodukte und für die Ausgangsund Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der allgemeinen Formel (I), in welchen eine Kombination der vorstehend als bevorzugt (vorzugsweise) aufgeführten Bedeutungen vorliegt.

Erfindungsgemäß besonders bevorzugt werden die Verbindungen der allgemeinen Formel (I), in welchen eine Kombination der vorstehend als besonders bevorzugt aufgeführten Bedeutungen vorliegt.

In den oben und nachstehend aufgeführten Restedefinitionen sind Kohlenwasserstoffreste, wie Alkyl oder Alkenyl - auch in Verbindungen mit Heteroatomen wie Alkoxy oder Alkylthio - soweit möglich jeweils geradkettig oder verzweigt.

Bevorzugte erfindungsgemäße Verbindungen sind Stoffe der Formeln (Ia) bis (Ie): 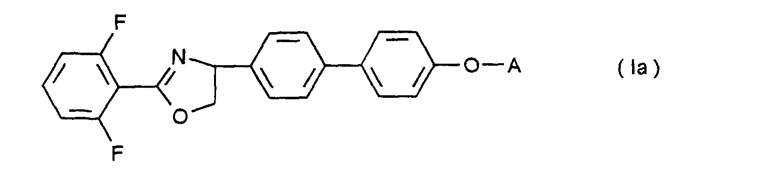 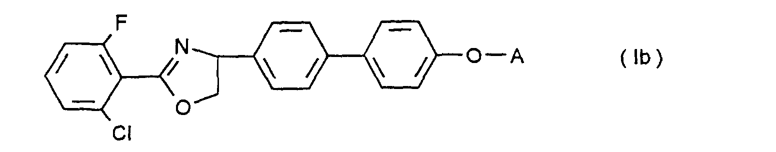 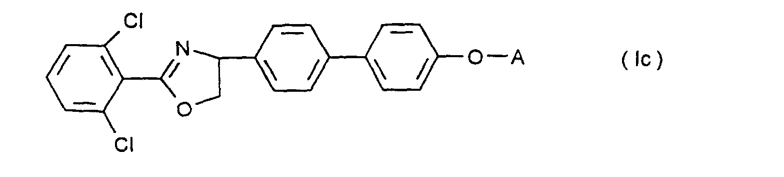 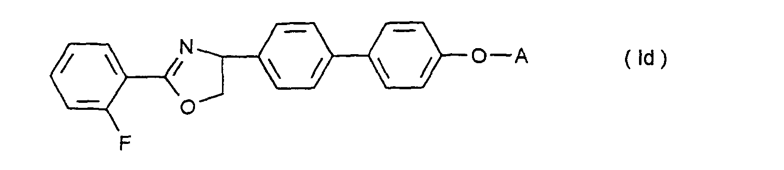 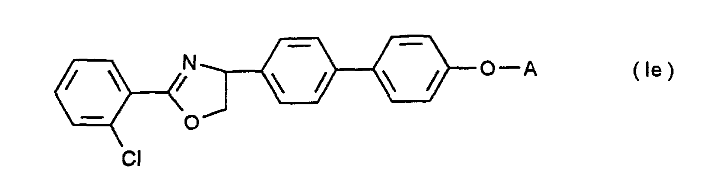 in welchen
- A: die in der Erfindungsdefinition genannten Bedeutungen hat.

Bevorzugte erfindungsgemäße Verbindungen sind auch Stoffe der Formel (IA): 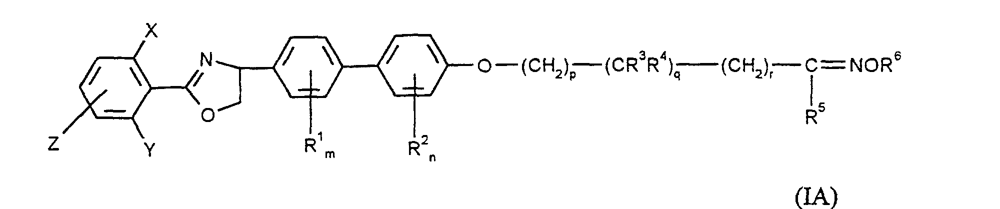 in welcher
- R¹, R², R³, R⁴, R⁵, R⁶, X, Y, Z, m, n, p, q und r: die in der Erfindungsdefinition genannten Bedeutungen haben, wobei unter diesen wiederum jene mit m = 0 und Z = Wasserstoff bevorzugt sind, besonders bevorzugt m = 0, n = 0 und Z = Wasserstoff.

Verwendet man zur Durchführung des erfindungsgemäßen Verfahrens beispielsweise 2-(2,6-Difluorphenyl)-4-(4'-hydroxibiphenyl-4-yl)-1,3-oxazolin und Benzoylmethylbromid als Ausgangsstoffe, so kann der Reaktionsablauf durch das folgende Formelschema wiedergegeben werden:

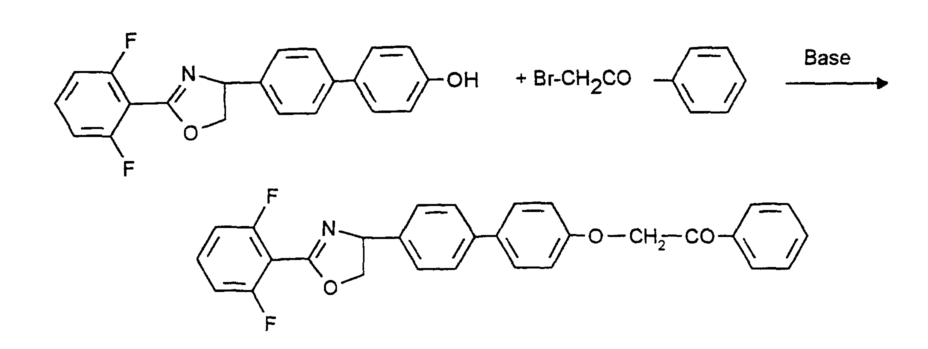

Die beim erfindungsgemäßen Verfahren als Ausgangsstoffe zu verwendenden Hydroxibiphenyloxazoline sind durch die Formel (II) allgemein definiert. In der Formel (II) haben X, Y, Z, R¹, R², m und n vorzugsweise bzw. besonders bevorzugt diejenigen Bedeutungen, die bereits oben im Zusammenhang mit der Beschreibung der erfindungsgemäßen Verbindungen der Formel (I) vorzugsweise bzw. besonders bevorzugt für diese Substituenten und Indizes angegeben wurden.

Die Hydroxibiphenyloxazoline der Formel (II) sind noch nicht bekannt. Sie sind jedoch teilweise Gegenstand eigener älterer Anmeldungen, die noch nicht zum Stand der Technik gehören (vgl. z.B. die Deutsche Patentanmeldung mit dem Aktenzeichen 44 44 108.1 vom 12.12.1994) und/oder können nach allgemein bekannten Verfahren erhalten werden (vgl. z.B. EP-A-0 432 661), indem man Amid-Derivate der Formel (IV) 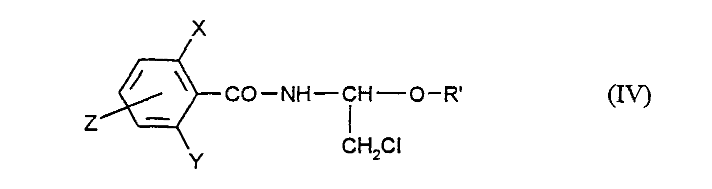 in welcher
- X, Y und Z: die oben angegebene Bedeutung haben und
- R': für C₁-C₄-Alkyl, vorzugsweise Methyl oder Ethyl steht,
mit Biphenyl-Derivaten der Formel (V) 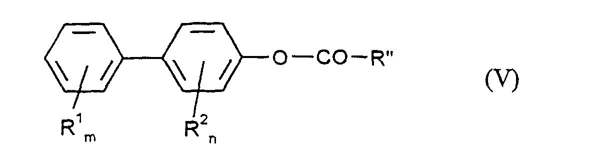 in welcher
- R¹, R², m und n: die oben angegebenen Bedeutungen haben und
- R": für C₁-C₄-Alkyl oder C₁-C₄-Alkoxy, vorzugsweise Methyl, Ethyl, Methoxy oder Ethoxy steht,
in Gegenwart eines sauren Katalysators, wie beispielsweise Schwefelsäure, Essigsäure oder Aluminiumchlorid und in Gegenwart eines Verdünnungsmittels, wie beispielsweise Methylenchlorid oder Acetonitril, bei Temperaturen zwischen 0°C und 80°C umsetzt;
die so erhaltenen Verbindungen der Formel (VI) 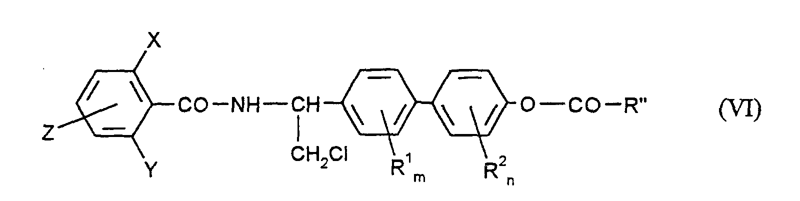 in welcher
- R¹, R², m, n, R", X, Y und Z: die oben angegebene Bedeutung haben,
in Gegenwart einer Base, wie beispielsweise Natronlauge, gegebenenfalls in Gegenwart eines Katalysators, wie beispielsweise Ammoniumverbindungen und gegebenenfalls in Gegenwart eines Verdünnungsmittels, wie beispielsweise Dimethylformamid, bei Temperaturen zwischen 0°C und 100°C zu Biphenyloxazolinen der Formel (VII) 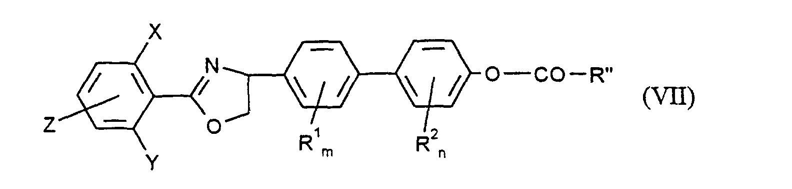 in welcher
- R¹, R², m, n, R", X, Y und Z: die oben angegebene Bedeutung haben,
cyclisiert und diese in üblicher Weise in Gegenwart eines Verdünnungsmittels, wie beispielsweise Methanol bei Raumtemperatur verseift, wie beispielsweise mittels wässriger Ammoniaklösung, wobei Cyclisierung und Verseifung gegebenenfalls auch in einer Eintropfreaktion erfolgen können.

Die Amid-Derivate der Formel (IV) sind bekannt (vgl. z.B. EP-A-0 594 179) und/oder können nach dort angegebenen Methoden erhalten werden.

Die außerdem beim erfindungsgemäßen Verfahren als Ausgangsstoffe zu verwendenden Verbindungen sind durch die Formel (III) allgemein definiert. In der Formel (III) hat A vorzugsweise bzw. besonders bevorzugt diejenigen Bedeutungen, die bereits oben im Zusammenhang mit der Beschreibung der erfindungsgemäßen Verbindungen der Formel (I) vorzugsweise bzw. besonders bevorzugt für diesen Substituenten angegeben wurden.

M steht für eine übliche Abgangsgruppe, vorzugsweise Halogen, insbesondere Chlor oder Brom; Alkylsulfonyloxy, insbesondere Methylsulfonyloxy; oder gegebenenfalls substituiertes Arylsulfonyloxy, insbesondere Phenylsulfonyloxy, p-Chlorphenylsulfonyloxy oder Tolylsulfonyloxy.

Die Verbindungen der Formel (III) sind allgemein bekannte Verbindungen der Organischen Chemie.

Als Verdünnungsmittel kommen zur Durchführung des erfindungsgemäßen Verfahrens alle üblichen Lösungsmittel in Frage. Vorzugsweise verwendbar sind gegebenenfalls halogenierte, aromatische oder aliphatische Kohlenwasserstoffe, Ketone, Nitrile und Amide. Genannt seien beispielsweise Toluol, Aceton, Acetonitril Dimethylfomamid und Dimethylacetamid.
Als Base kommen zur Durchführung des erfindungsgemäßen Verfahrens alle üblichen anorganischen und organischen Basen in Frage. Genannt seien beispielsweise tertiäre Amine wie Triethylamin, DBN (Diazabicyclononen), DBU (Diazabicycloundecen), DABCO (Diazabicyclooctan), Alkali- und Erdalkalihydroxide wie z.B. Natriumhydroxid, Kaliumhydroxid und Calciumhydroxid sowie Alkali- und Erdalkalicarbonate wie z.B. Natriumcarbonat oder Kaliumcarbonat.

Das erfindungsgemäße Verfahren wird gegebenenfalls in Gegenwart eines Phasentransferkatalysators durchgeführt. Beispielhaft genannt seien quartäre Ammoniumsalze, wie Tetraoctylammoniumbromid oder Benzyltriethylammoniumchlorid sowie Tris(3,6-dioxaheptyl)amin (TDA).

Die Reaktionstemperaturen können bei der Durchführung des erfindungsgemäßen Verfahrens in einem größeren Bereich variiert werden. Im allgemeinen arbeitet man bei Temperaturen zwischen -20°C und 100°C, bevorzugt zwischen 0°C und 60°C.

Bei der Durchführung des erfindungsgemäßen Verfahrens arbeitet man im allgemeinen in angenähert äquimolaren Mengen. Man kann jedoch auch einen Überschuß der Verbindung der Formel (III) verwenden.

Die Aufarbeitung und Isolierung erfolgen in allgemein üblicher Art und Weise.

In manchen Fällen erweist es sich als vorteilhaft, Verbindungen der Formel (I), in denen R für eine der Gruppierungen (e) bis (k) steht, durch allgemein übliche und bekannte Derivatisierungen der entsprechenden Keto-Derivate, Carbonsäure-Derivate und Nitrile, d.h. Verbindungen der Formel (I), in denen R für Cyano oder eine der Gruppierungen (a) bis (d) steht, zu erhalten.

Besonders bevorzugt sind hierbei die Derivatisierungen, die zu Ketalen, Thioketalen, Oximen, Oximethern oder Hydrazonen führen.

Die erfindungsgemäßen Verbindungen der Formel (I) können auch erhalten werden, indem man
A) in einer ersten Stufe Verbindungen der Formel (VIII) 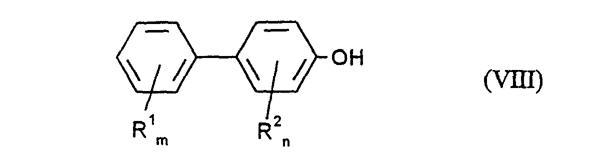 in welcher
   - R¹, R², m und n: die oben angegebene Bedeutung haben,
   mit einer Verbindung der Formel (III)

   M-A (III)

   in welcher
   - M und A: die oben angegebene Bedeutung haben,
   unter den Bedingungen des erfindungsgemäßen Verfahrens umsetzt;
B) in einer zweiten Stufe die so erhaltenen Verbindungen der Formel (IX) 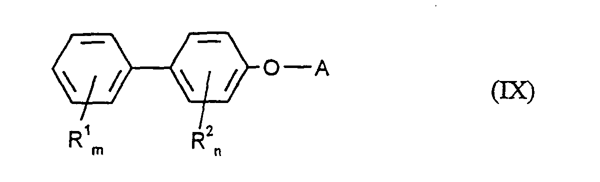 in welcher
   - A, R¹, R², m und n: die oben angegebene Bedeutung haben,
   mit Acetylchlorid in Gegenwart eines Verdünnungsmittels wie z.B. Methylenchlorid oder Dichlorethan und in Gegenwart einer für Friedel-Crafts-Reaktionen geeigneten Säure bzw. Lewis-Säure, wie z.B. Tetrafluorborsäure oder Aluminiumchlorid bei Temperaturen zwischen -20°C und +50°C umsetzt;
C) in einer dritten Stufe die so erhaltenen Verbindungen der Formel (X) 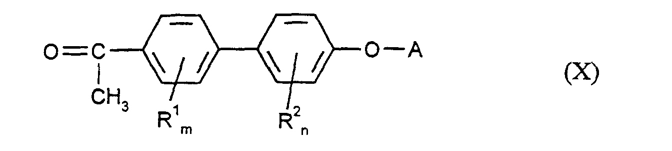 in welcher
   - A, R¹, R², m und n: die oben angegebene Bedeutung haben,
   gegebenenfalls in Gegenwart eines Verdünnungsmittels, wie z.B. Methylenchlorid oder Tetrachlorkohlenstoff bei Temperaturen zwischen -10°C und 25°C chloriert oder bromiert;
D) in einer vierten Stufe die so erhaltenen Verbindungen der Formel (XI) 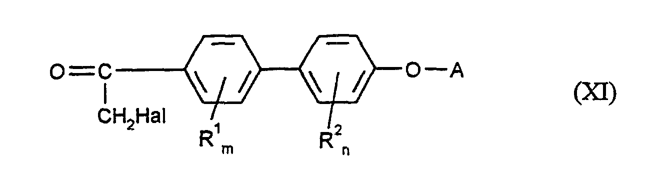 in welcher
   - A, R¹, R², m und n: die oben angegebene Bedeutung haben und
   - Hal: für Chlor oder Brom steht,
   mit einem Salz der Ameisensäure, wie z.B. Natriumformiat; in Gegenwart eines Verdünnungsmittels, gegebenenfalls im Gemisch mit Wasser, wie z.B. Ethanol/Wasser und gegebenenfalls in Gegenwart eines Phasentransferkatalysators, wie z.B. quartiären Ammoniumsalzen bei Temperaturen zwischen 50°C und 150°C umsetzt;
E) in einer fünften Stufe die so erhaltenen Verbindungen der Formel (XII) 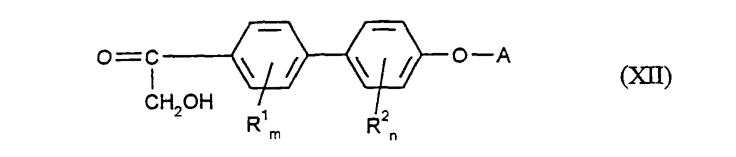 in welcher
   - A, R¹, R², m und n: die oben angegebene Bedeutung haben,
   mit O-Methylhydroxylamin, gegebenenfalls in Form eines Salzes, z.B. des Hydrochlorids, in Gegenwart eines Verdünnungsmittels, z.B. Alkoholen oder Ethern, und gegebenenfalls in Gegenwart einer Base, z.B. Natriumacetat, bei Temperaturen zwischen 0°C und 60°C umsetzt;
F) in einer sechsten Stufe die so erhaltenen Verbindungen der Formel (XIII) 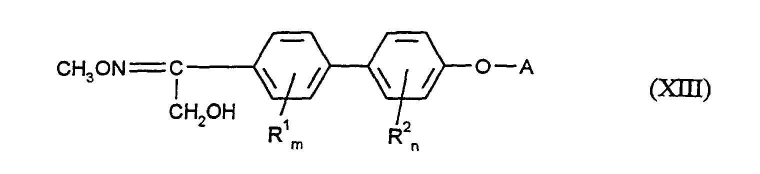 in welcher
   - A, R¹, R², m und n: die oben angegebene Bedeutung haben,
   mit einem Reduktionsmittel, wie z.B. Natriumboranat, in Gegenwart einer Säure, wie z.B. Trifluoressigsäure und gegebenenfalls in Gegenwart eines Verdünnungsmittels, wie z.B. Tetrahydrofuran bei Temperaturen zwischen 0°C und 120°C umsetzt;
G) in einer siebten Stufe die so erhaltenen Verbindungen der Formel (XIV) 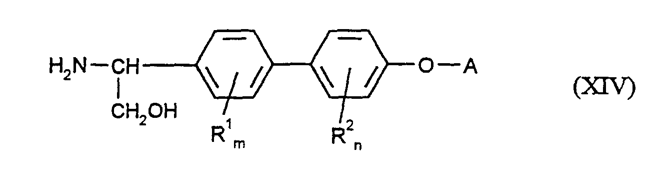 in welcher
   - A, R¹, R², m und n: die oben angegebene Bedeutung haben,
   mit Benzoylchloriden der Formel (XV) 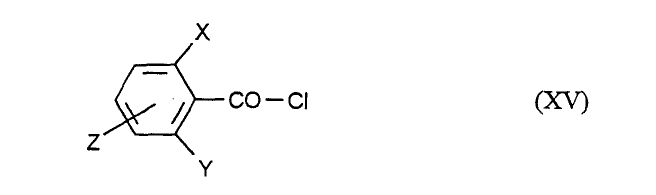 in welcher
   - X, Y und Z: die oben angegebene Bedeutung haben,
   gegebenenfalls in Gegenwart einer Base, wie z.B. Triethylamin und gegebenenfalls in Gegenwart eines Verdünnungsmittels, wie z.B. Tetrahydrofuran bei Temperaturen zwischen 0°C und 100°C umsetzt;
H) in einer achten Stufe die so erhaltenen Verbindungen der Formel (XVI) 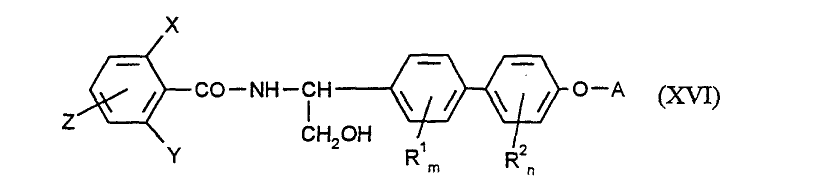 in welcher
   - A, R¹, R², m, n, X, Y und Z: die oben angegebene Bedeutung haben,
   mit einem Chlorierungsmittel, wie z.B. Thionylchlorid, gegebenenfalls in Gegenwart eines Verdünnungsmittels, wie z.B. Toluol bei Temperaturen zwischen 20°C und 100°C umsetzt;
I) in einer neunten Stufe die so erhaltenen Verbindungen der Formel (XVII) 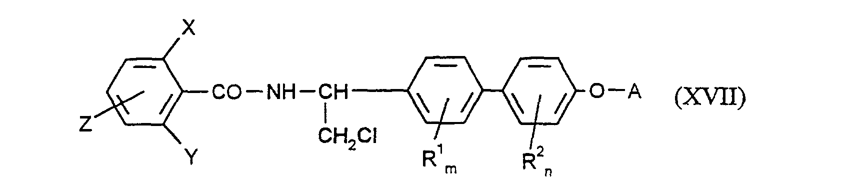 in welcher
   - A, R¹, R², m, n, X, Y und Z: die oben angegebene Bedeutung haben,
   in Gegenwart einer Base, wie z.B. Natronlauge; gegebenenfalls in Gegenwart eines Phasentransferkatalysators, wie z.B. Ammoniumverbindungen und gegebenenfalls in Gegenwart eines Verdünnungsmittels, wie z.B. Dimethylformamid bei Temperaturen zwischen 0°C und 100°C zu den erfindungsgemäßen Verbindungen der Formel (I) cyclisiert.

Die Verbindungen der Formel (XVII) können auch direkt erhalten werden, indem man Amid-Derivate der Formel (IV) mit Verbindungen der Formel (IX) in Gegenwart eines sauren Katalysators, wie z.B. Fluorwasserstoff, Bortrifluorid oder Aluminiumchlorid und in Gegenwart eines Verdünnungsmittels, wie z.B. Methylenchlorid oder Acetonitril bei Temperaturen zwischen 0°C und 80°C umsetzt.

Die Ausgangsstoffe der Formel (VIII) sind bekannt und/oder lassen sich nach bekannten Methoden in einfacher Weise herstellen.

Man erhält die Verbindungen der Formel (VIII) beispielsweise, indem man gegebenenfalls substituierte Biphenyle sulfoniert und dann mit Alkalihydroxiden zu den Hydroxybiphenylen umsetzt oder Aminobiphenyle diazotiert und verkocht. (vgl. z.B. Houben-Weyl, Band VI/lc (1976), Seite 216 und 251).

Die Benzoylchloride der Formel (XV) sind allgemein bekannte Verbindungen der Organischen Chemie.

Die als Zwischenprodukte auftretenden Verbindungen der Formeln (X), (XI), (XII), (XIII), (XIV), (XVI) und (XVII) sind noch nicht bekannt und sind auch Gegenstand der Erfindung.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorratsund Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formel (I) zeichnen sich insbesondere durch eine hohe insektizide und akarizide Wirksamkeit aus.

Sie lassen sich mit besonders gutem Erfolg zur Bekämpfung von pflanzenschädigenden Insekten, wie beispielsweise gegen die Meerretüchblattkäfer-Larven (Phaedon cochlaeriae), die Raupen der Kohlschabe (Plutella maculipennis), die Pfirsichblattlaus (Mycus persicae) und die Raupen des Eulenfalters (Spodoptera frugiperda) oder zur Bekämpfung von pflanzenschädigenden Milben, wie beispielsweise gegen die gemeine Spinnmilbe (Tetranychus urticae) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygieneund Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine gute entwicklungshemmende Wirkung gegen Fliegenlarven von Lucilla cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die Herstellung und die Verwendung der erfindungsgemäßen Wirkstoffe gehen aus den nachfolgenden Beispielen hervor.

### Herstellungsbeispiele:

### Beispiel 1

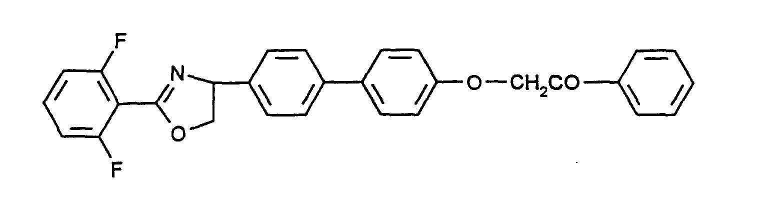

3,51 g (0,01 Mol) 2-(2,6-Difluorphenyl)-4-(4'-hydroxy-biphenyl-4-yl)-1,3-oxazolin, 2,0 g (0,01 Mol) Benzoylmethylbromid und 1,38 g (0,01 Mol) Kaliumcarbonat werden in 50 ml Acetonitril suspendiert. Nach Zugabe von drei Tropfen Tris(3,6-dioxaheptyl)amin (TDA) wird das Reaktionsgemisch 18 Stunden bei 80°C gerührt und anschließend eingeengt. Der Rückstand wird in 100 ml Methylenchlorid aufgenommen, mit Wasser gewaschen, über Natriumsulfat getrocknet, eingeengt und säulenchromatographisch mit Methylenchlorid als Laufmittel gereinigt.

Man erhält 1,7 g (36,2 % der Theorie) 2-(2,6-Difluorphenyl)-4-(4'-benzoylmethoxy-biphenyl-4-yl)-1,3-oxazolin vom Schmelzpunkt Fp.: 78-82°C.

### Herstellung des Ausgangsproduktes

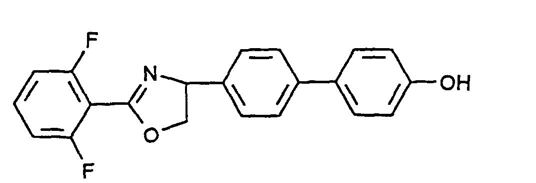

10,9 g (0,026 mol) 2-(2,6-Difluorphenyl)-4-(4'-ethoxycarbonyloxybiphenyl-4-yl)-1,3-oxazolin werden in 50 ml Methanol suspendiert, dann werden bei Raumtemperatur 35,4 ml (0,52 mol) 25 %-ige wässrige Ammoniaklösung zugetropft. Nach 28 Stunden bei Raumtemperatur wird der Niederschlag abgesaugt und mit wenig Methanol gewaschen.
Ausbeute 7,6 g (97,4 % der Theorie), Fp.: 180 bis 182°C.

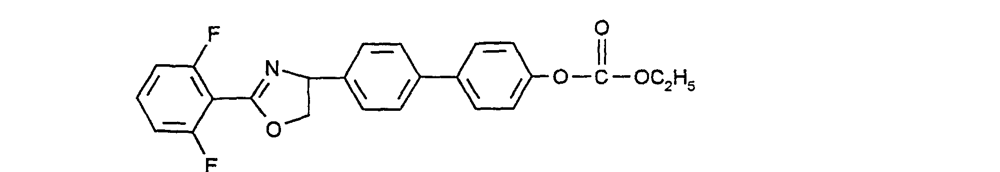

Zu 19,8 g (0,043 mol) 2-(2,6-Difluorbenzoylamido)-2-(4'-ethoxycarbonyloxybiphenyl-4-yl)-1-chlorethan in 120 ml Dimethylformamid werden bei 5°C 4 ml (0,045 mol) 50 %-ige Natronlauge getropft. Nach 2 Stunden bei Raumtemperatur rührt man in 500 ml Eiswasser ein und saugt die ausgefallenen Kristalle ab.
Ausbeute: 15,1 g (83 % der Theorie), Fp.: 98 bis 100°C.

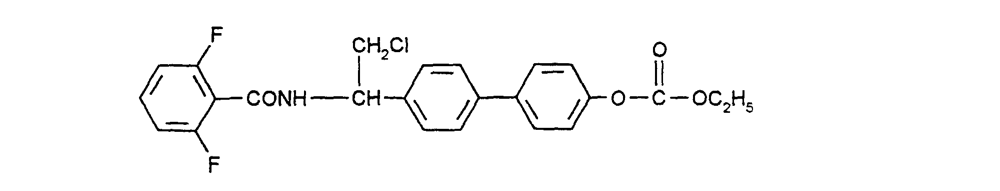

In das Gemisch von 53 g (0,213 mol) 2-(2,6-Difluorbenzoylamido)-2-methoxy-1-chlorethan, 48,4 g (0,2 mol) 4-Ethoxycarbonyloxybiphenyl, und 12 ml Eisessig in 200 ml Methylenchlorid werden bei 5°C innerhalb von 30 Minuten 130,4 g (0,98 mol) Aluminiumchlorid portionsweise eingetragen. Dabei verfärbt sich der Ansatz über blau nach rotviolett. Man rührt eine Stunde bei 5°C und 1 Stunde bei 10°C, gibt das Reaktionsgemisch vorsichtig auf Eis, dekantiert die Suspension vorsichtig von Wasser ab, engt am Rotationsverdampfer ein, versetzt den Rückstand mit 50 ml Acetonitril und saugt die ausgefallenen Kristalle ab. Ausbeute 42,8 g (47 % der Theorie), Fp.: 209°C.

### Beispiel 2

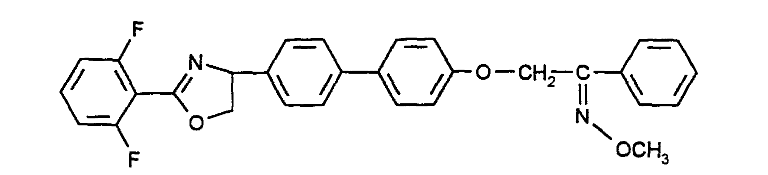

Zu 0,96 g (0,002 Mol) 2-(2,6-Difluorphenyl)-4-(4'-benzoylmethoxy-biphenyl-4-yl)-1,3-oxazolin (Bsp. 1) in 20 ml Methanol gibt man 0,167 g (0,002 Mol) O-Methylhydroxylamin-hydrochlorid und 0,164 g (0,002 Mol) Natriumacetat. Das Reaktionsgemisch wird 6 Stunden bei 65°C gerührt und danach eingeengt. Der Rückstand wird in 20 ml Methylenchlorid aufgenommen, mit Wasser gewaschen, über Natriumsulfat getrocknet und eingeengt. Der ölige Rückstand wird säulenchromatographisch gereinigt (Laufmittel: Toluol/Ether: 8/2).
Man erhält 0,7 g (70 % der Theorie) 2-(2,6-Difluorphenyl)-4-[4'-(2-methoximino-2-phenyl)-ethoxy-biphenyl-4]-4-yl)-1,3-oxazolin mit einem log p = 5,01.
- [log p =: Logarithmus des Verteilungskoeffizienten p der Substanz zwischen den Lösungsmitteln Octanol und Wasser, experimentell ermittelt aus reversed phase HPLC]

### Beispiel 3

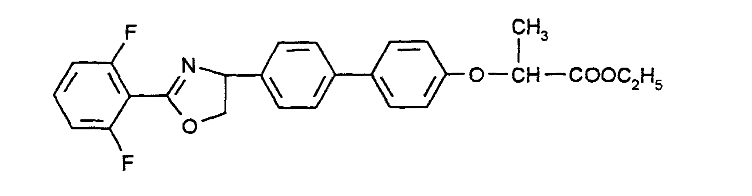

3,51 g (0,01 Mol) 2-(2,6-Difluorphenyl)-4-(4'-hydroxybiphenyl-4-yl)-1,3-oxazolin und 1,38 g (0,01 Mol) Kaliumcarbonat werden in 50 ml Acetonitril suspendiert und nach Zugabe von drei Tropfen Tris(3,6-dioxaheptyl)amin (TDA) mit 1,8 g (0,01 Mol) 2-Brompropionsäureethylester versetzt. Das Reaktionsgemisch wird 18 Stunden bei 65°C gerührt und anschließend eingeengt. Der Rückstand wird in 100 ml Methylenchlorid aufgenommen, mit Wasser gewaschen, über Natriumsulfat getrocknet und eingeengt. Der ölige Rückstand wird säulenchromatographisch mit Methylenchlorid als Laufmittel gereinigt.
Man erhält 2,5 g (55,4 % der Theorie) 2-(2,6-Difluorphenyl)-4-[4'-(1-ethoxycarbonyl-eth-1-yl-oxy)-biphenyl-4-yl]-1,3-oxazolin mit einem log p = 4,12.

### Beispiel 4

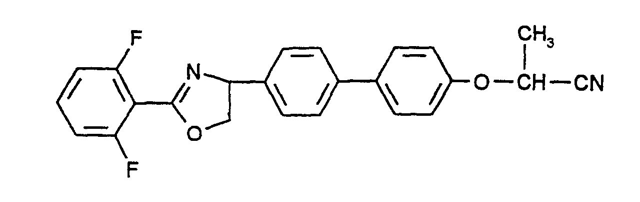

3,51 g (0,01 Mol) 2-(2,6-Difluorphenyl)-4-(4'-hydroxybiphenyl-4-yl)-1,3-oxazolin und 1,38 g (0,01 Mol) Kaliumcarbonat werden in 50 ml Acetonitril suspendiert und nach Zugabe von drei Tropfen Tris(3,6-dioxaheptyl)amin (TDA) mit 0,9 g (0,01 Mol) 2-Chlorpropionitril versetzt. Das Reaktionsgemisch wird 20 Stunden bei 80°C gerührt und anschließend eingeengt. Der Rückstand wird in 100 ml Methylenchlorid aufgenommen, mit Wasser gewaschen, über Natriumsulfat getrocknet und eingeengt. Der ölige Rückstand wird säulenchromatographisch mit Methylenchlorid als Laufmittel gereinigt.
Man erhält 2,6 g (64,4 % der Theorie) 2-(2,6-Difluorphenyl)-4-[4'-(2-propionitriloxy)-biphenyl-4-yl]-1,3-oxazolin vom Schmelzpunkt 100 - 120°C.

### Beispiel 5

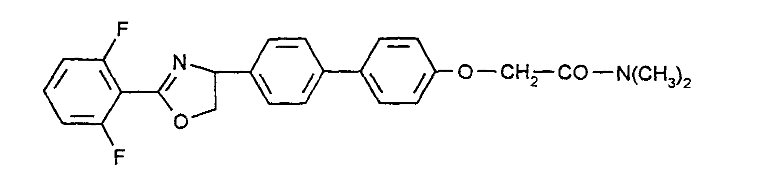

3,51 g (0,01 Mol) 2-(2,6-Difluorphenyl)-4-(4'-hydroxybiphenyl-4-yl)-1,3-oxazolin und 1,38 g (0,01 Mol) Kaliumcarbonat werden in 50 ml Acetonitril suspendiert und nach Zugabe von drei Tropfen Tris(3,6-dioxaheptyl)amin (TDA) mit 1,2 g (0,01 Mol) 2-Chlor-N,N-dimethyl-acetamid versetzt. Das Reaktionsgemisch wird 20 Stunden bei 80°C gerührt und anschließend eingeengt. Der Rückstand wird in 100 ml Methylenchlorid aufgenommen, mit Wasser gewaschen, über Natriumsulfat getrocknet und eingeengt. Der Rückstand wird mit Diisopropylether verrührt und eingeengt.
Man erhält 2,4 g (55 % der Theorie) 2-(2,6-Difluorphenyl)-4-(4'-dimethylaminocarbonylmethyloxy-biphenyl-4-yl)-1,3-oxazolin vom Schmelzpunkt 78-80°C.

Analog den Beispielen 1 bis 5 bzw. gemäß den allgemeinen Angaben zur Herstellung werden die in der nachfolgenden Tabelle angegebenen Verbindungen der Formel (I) erhalten:

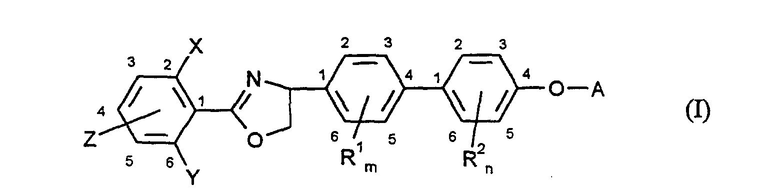

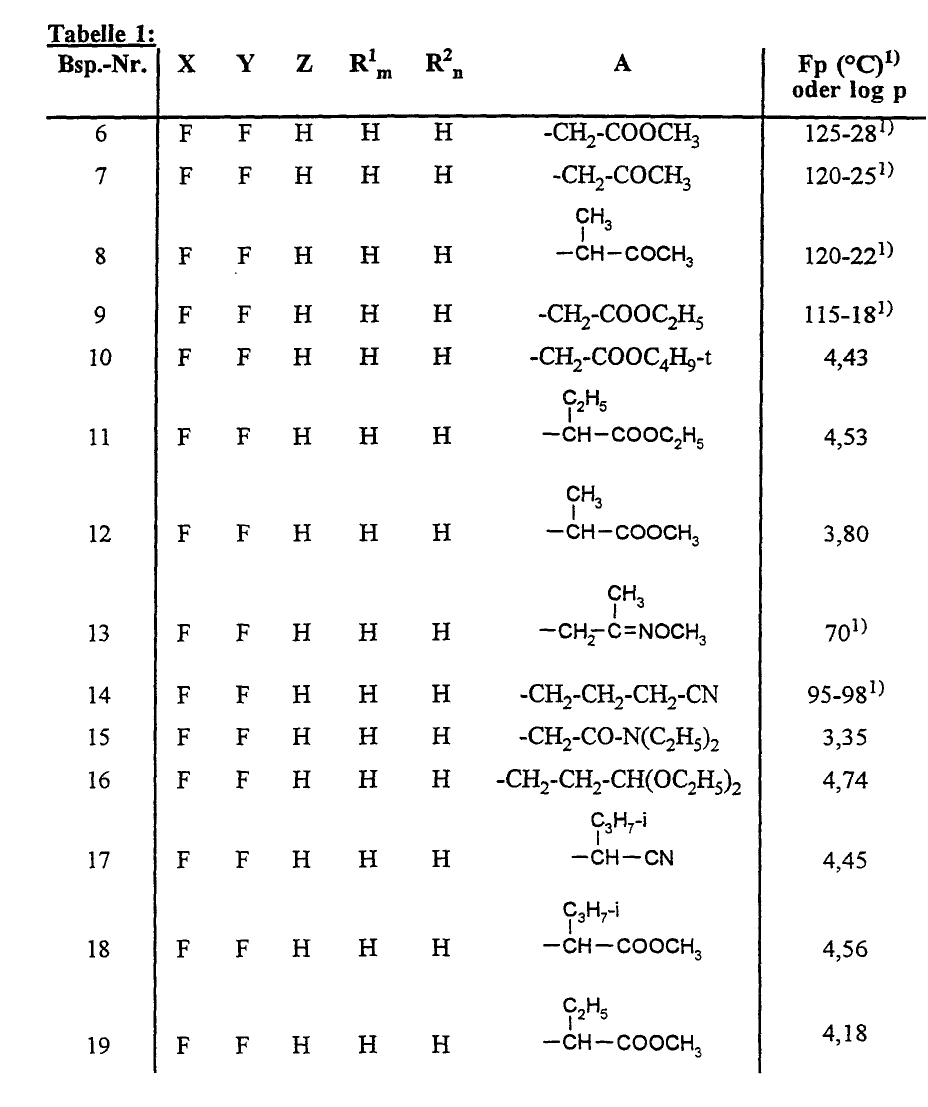

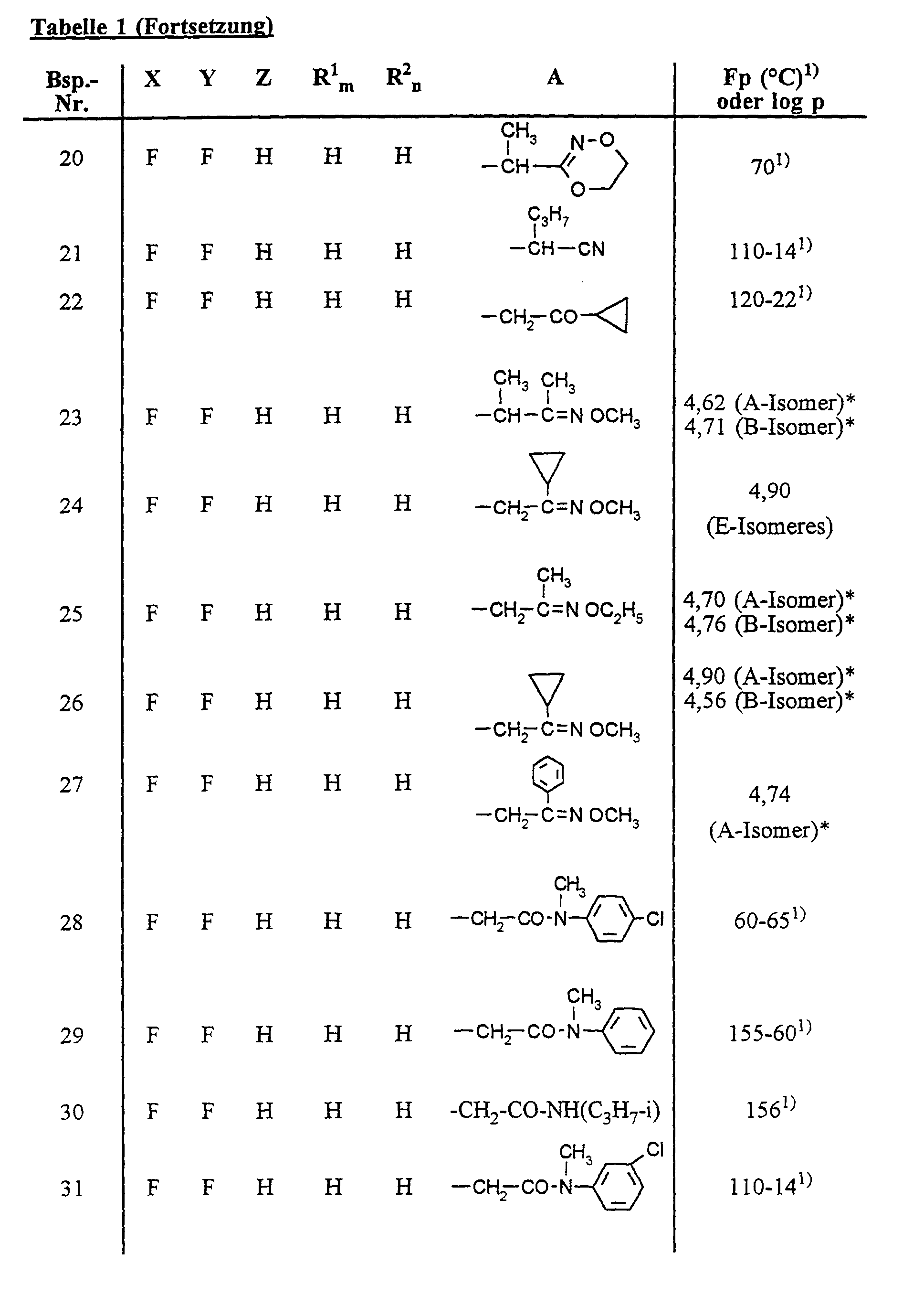

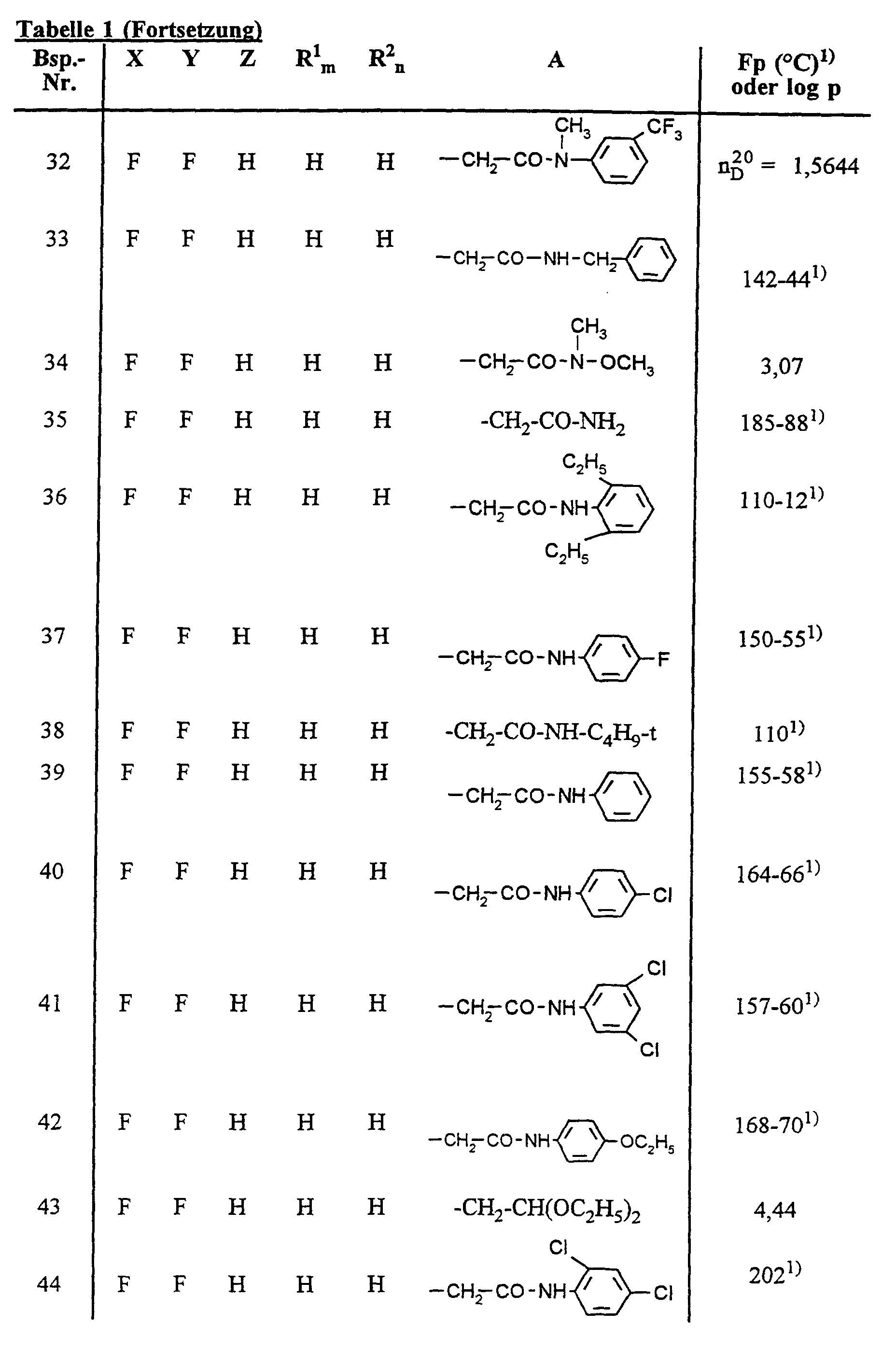

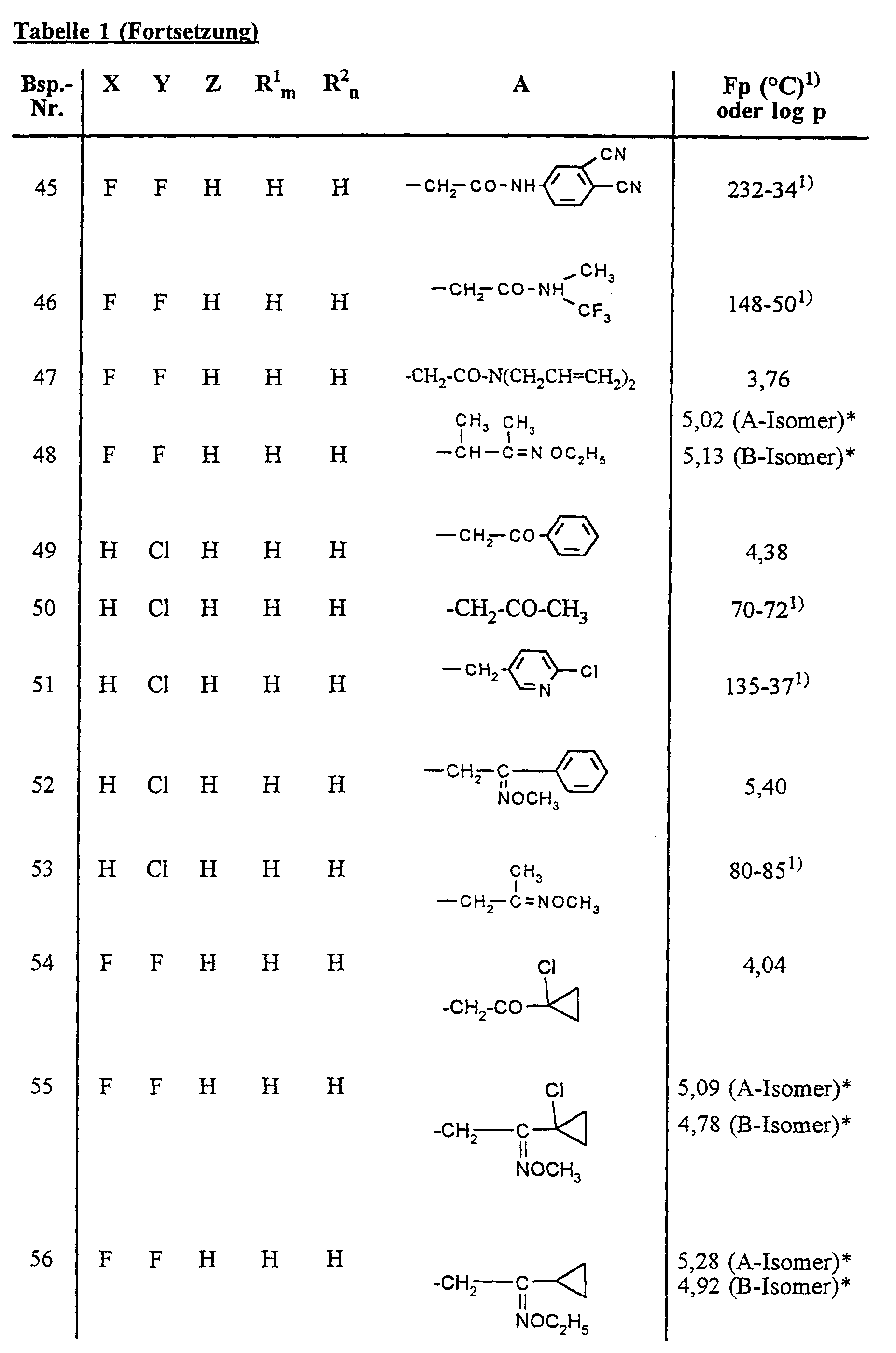

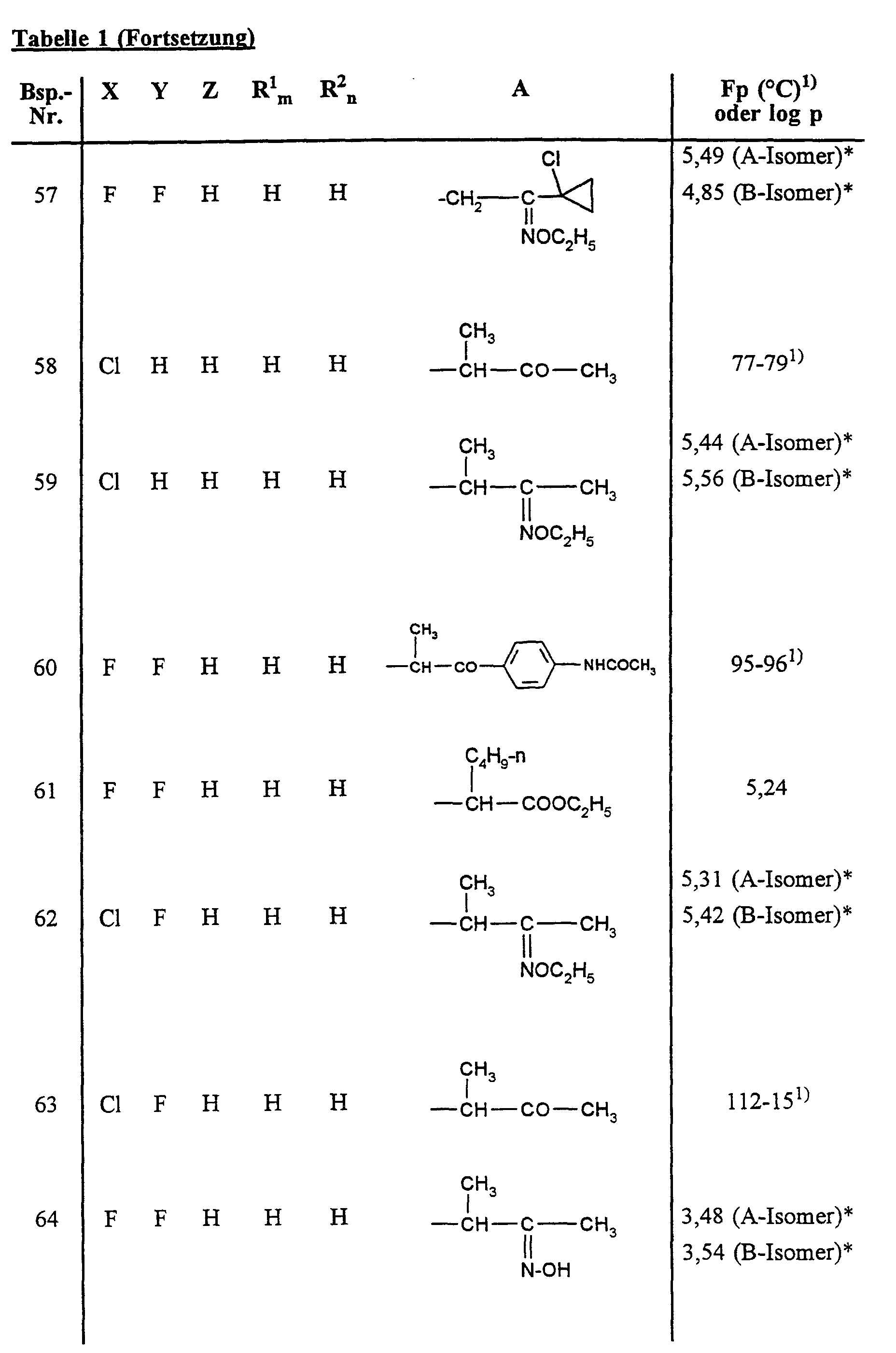

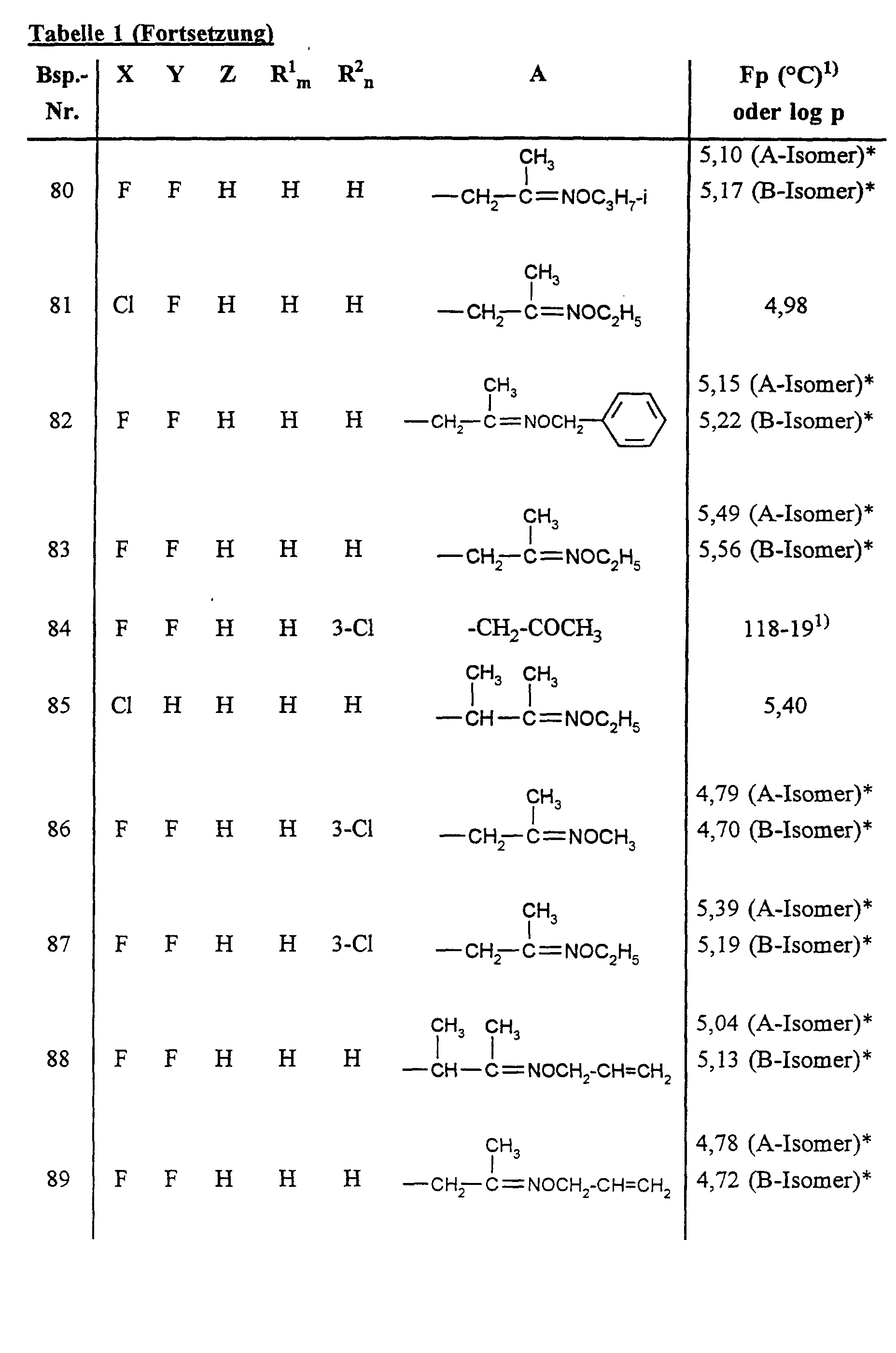

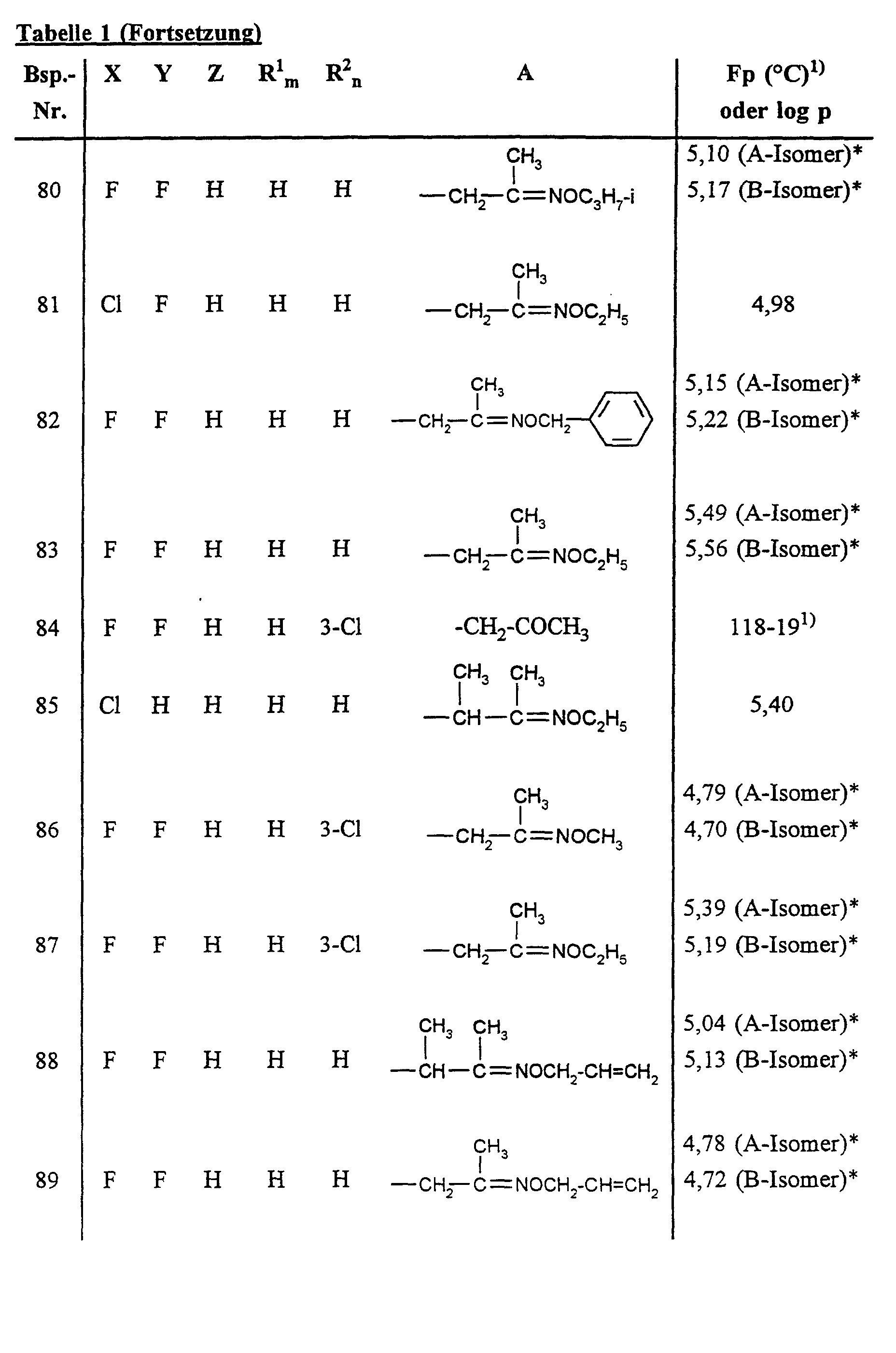

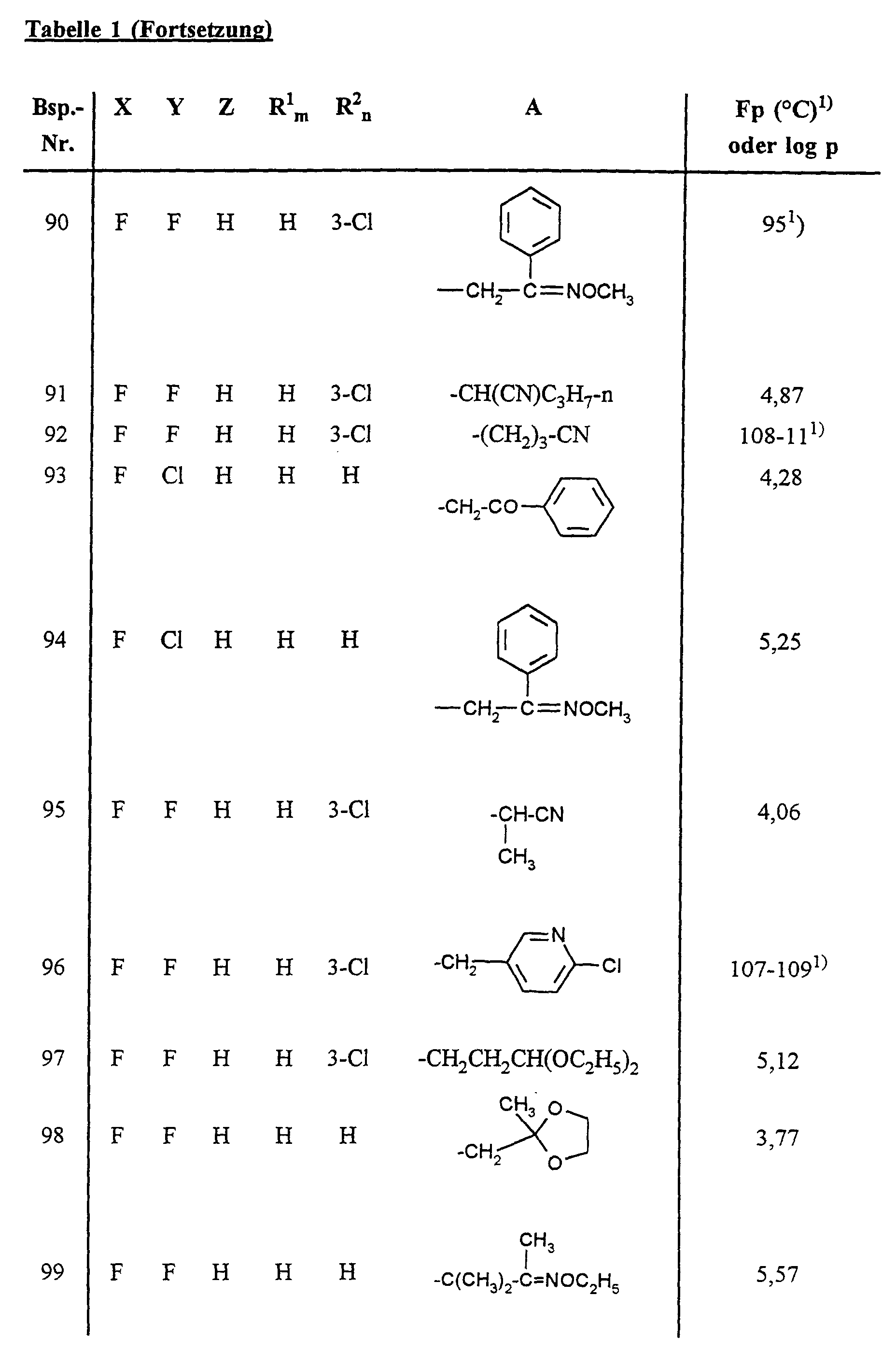

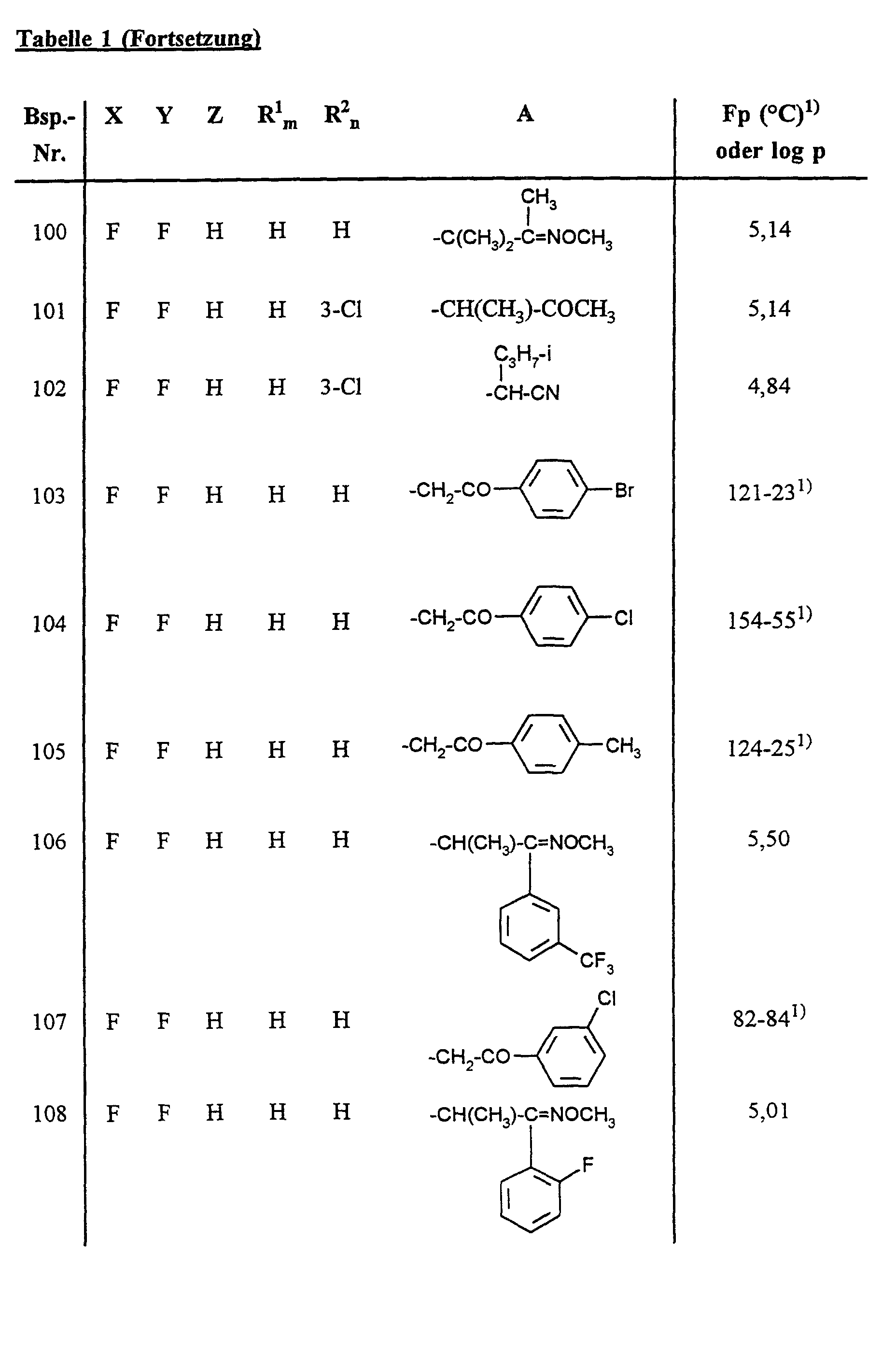

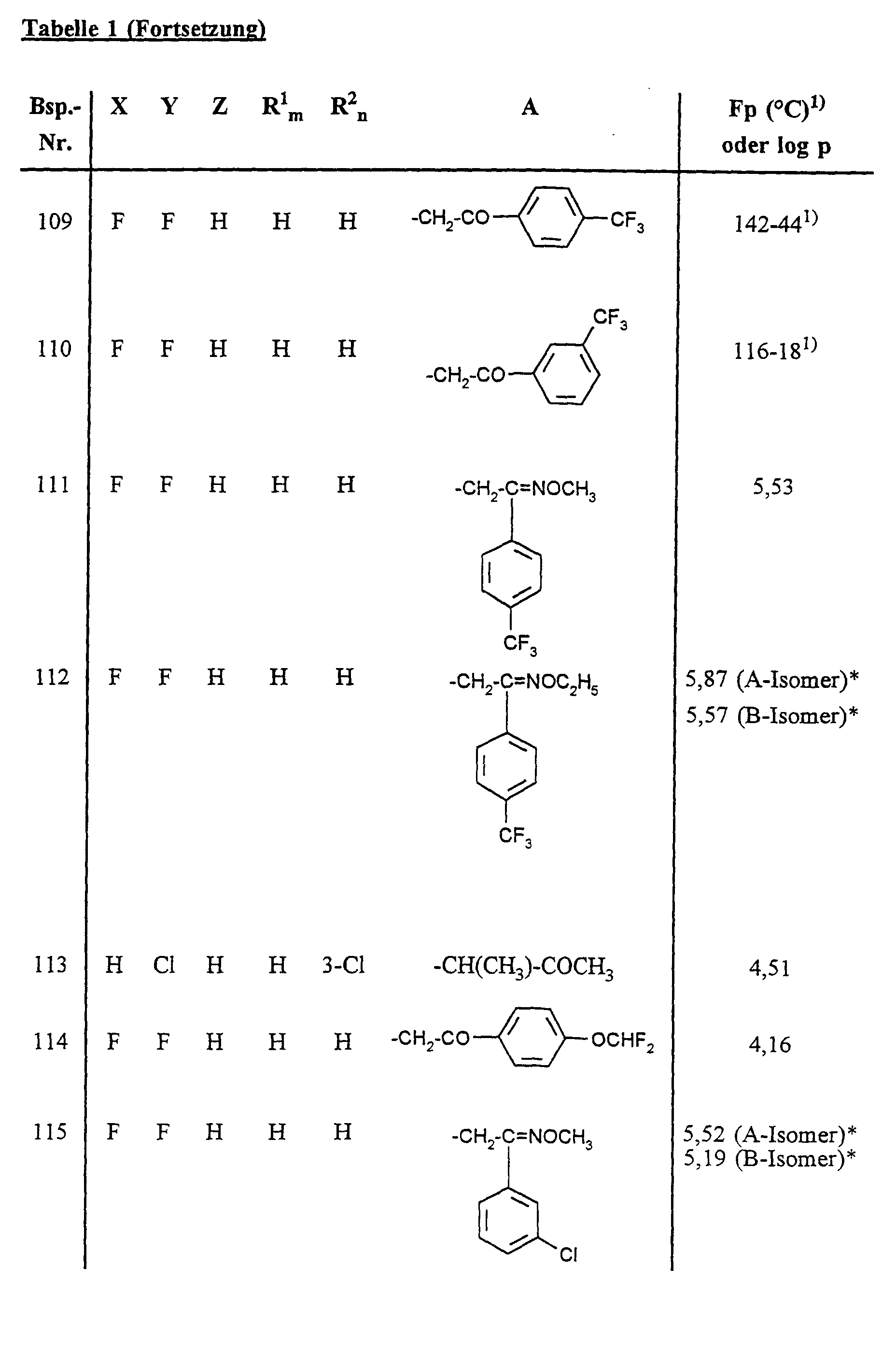

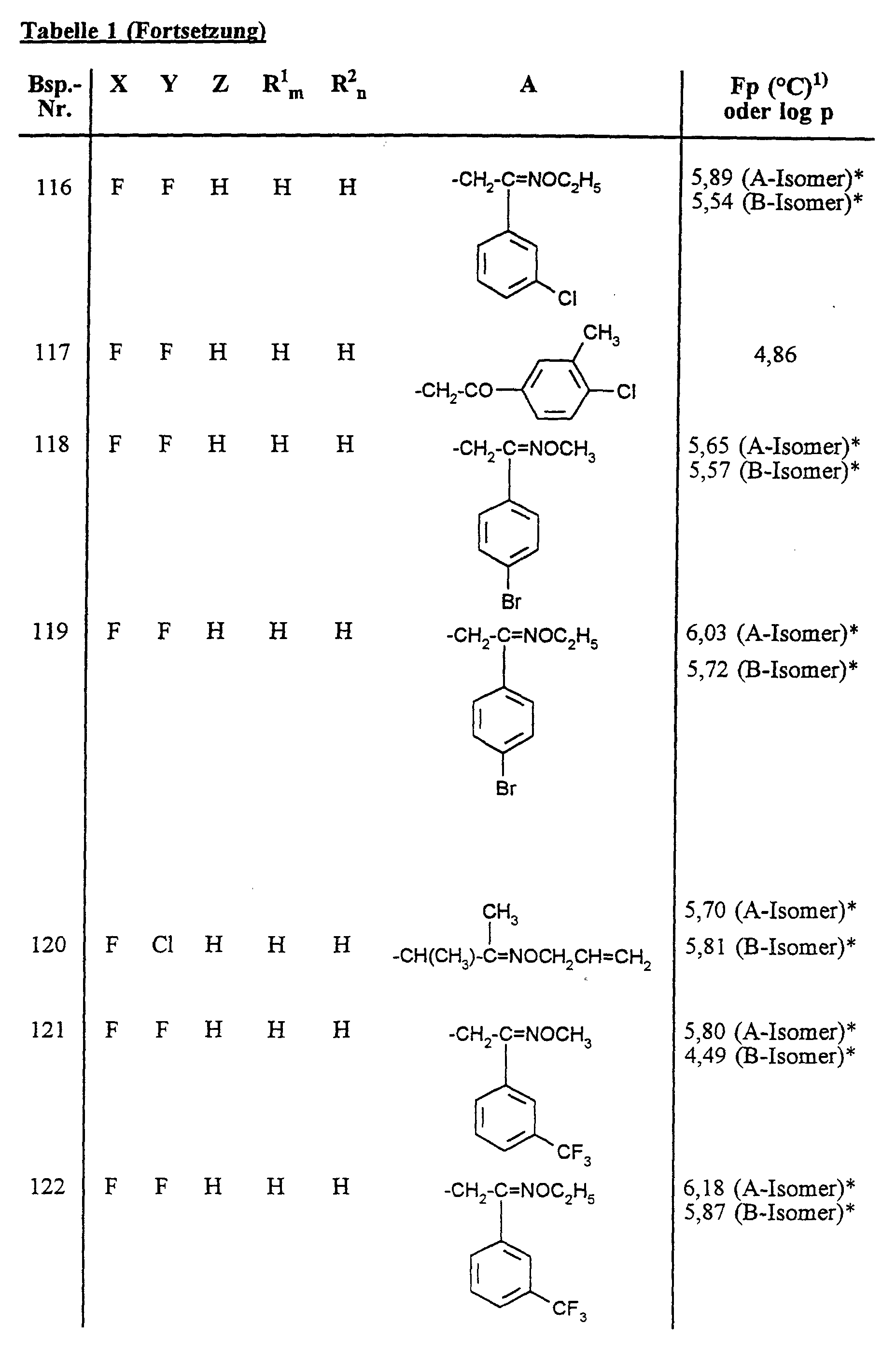

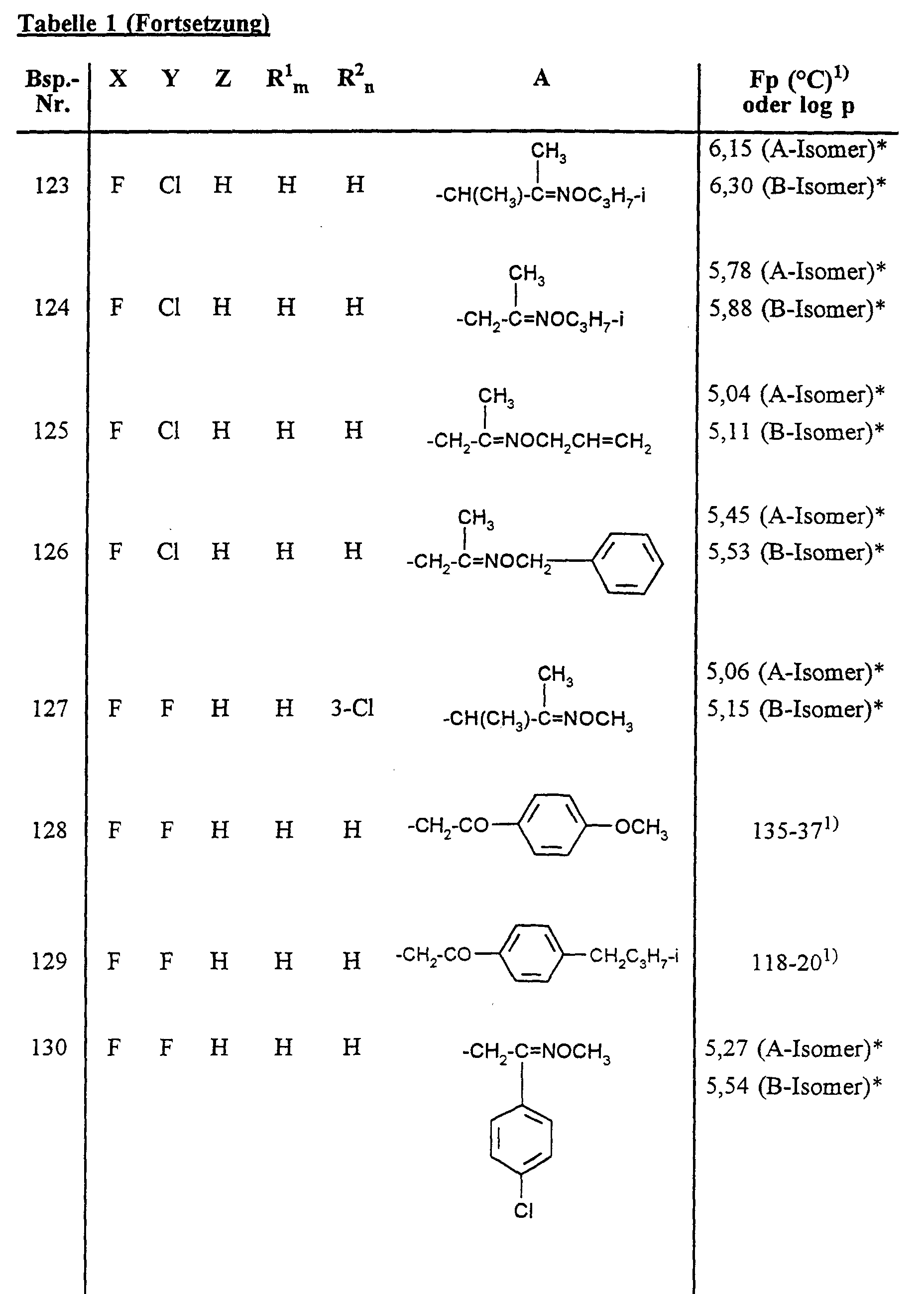

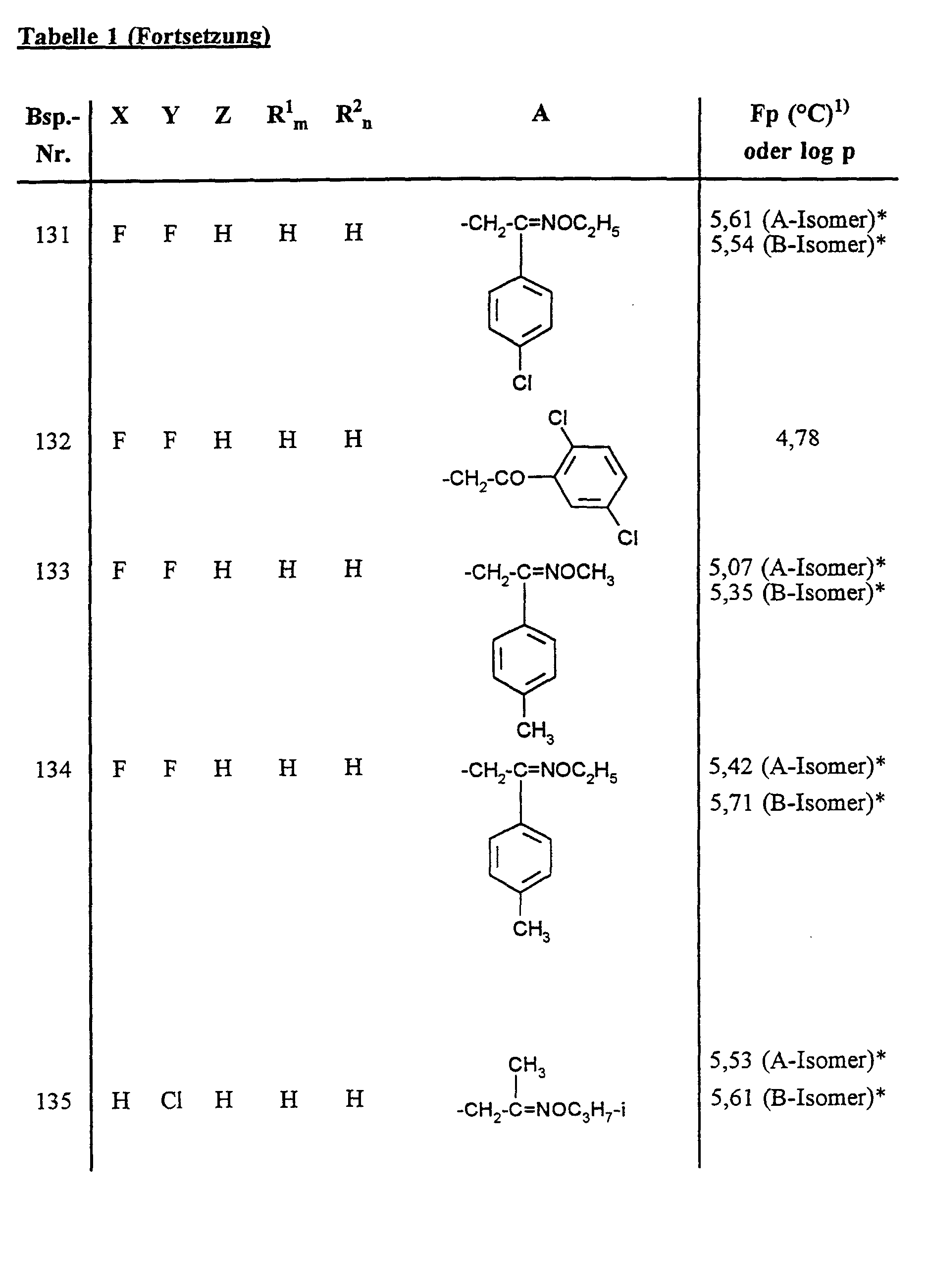

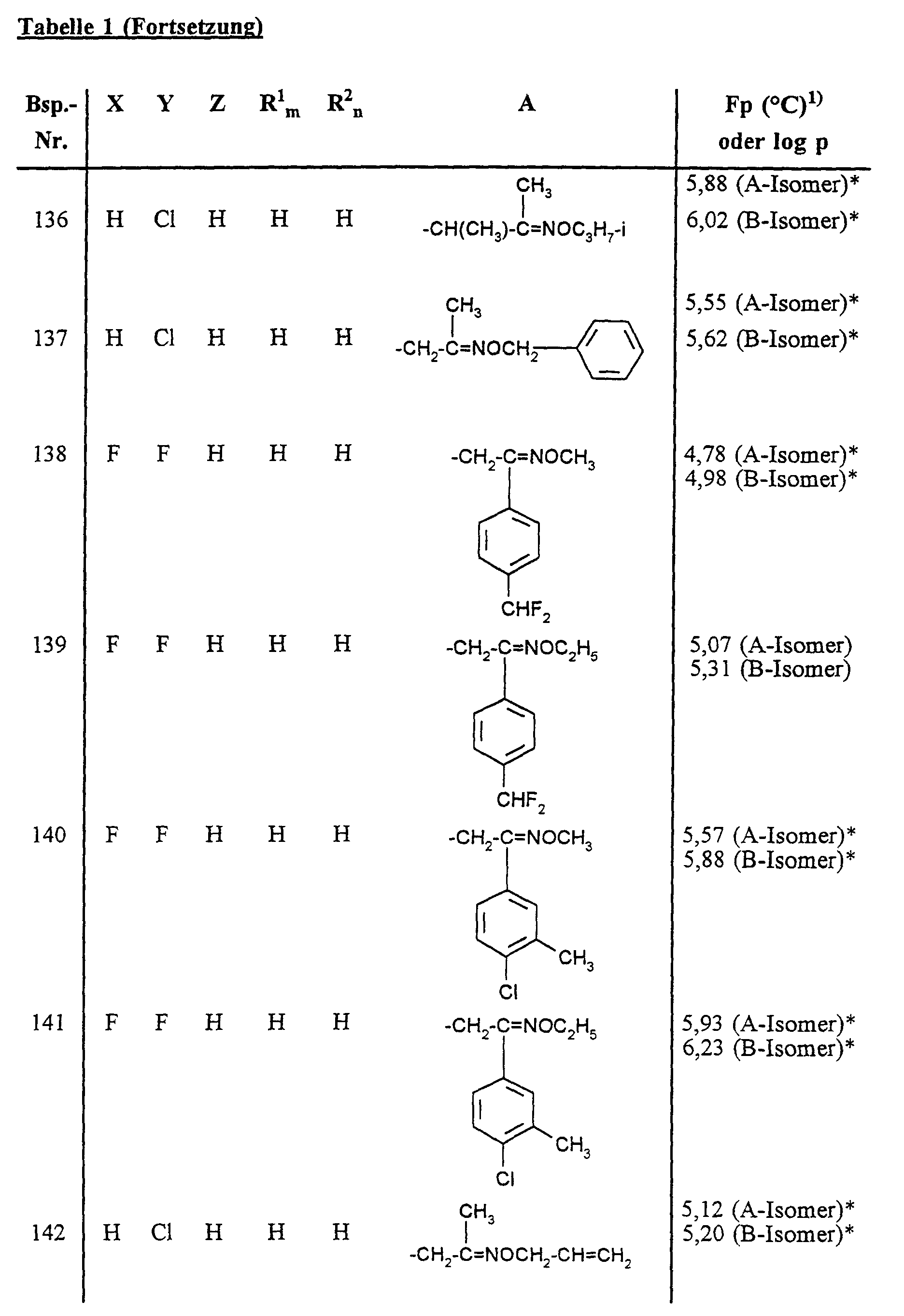

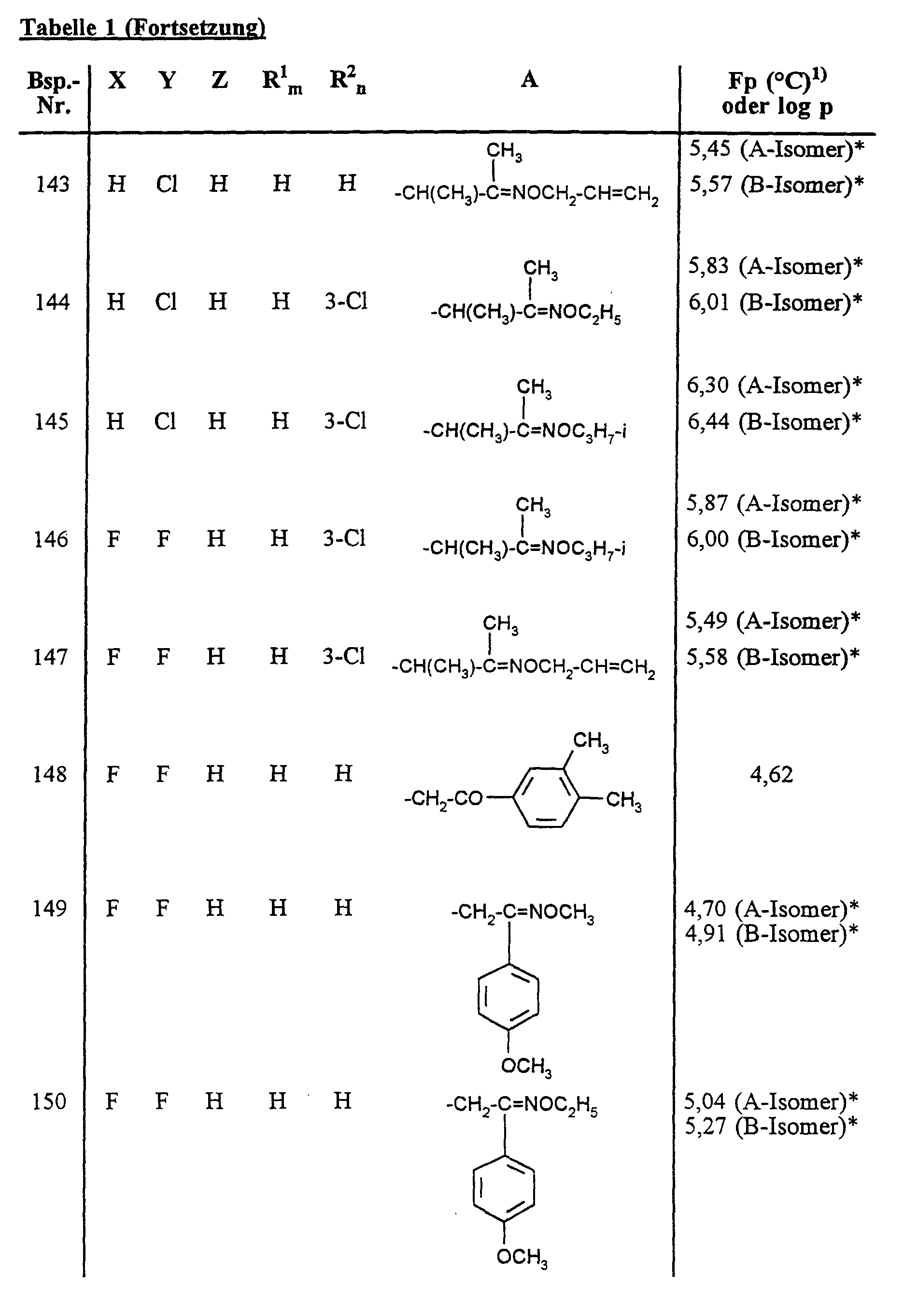

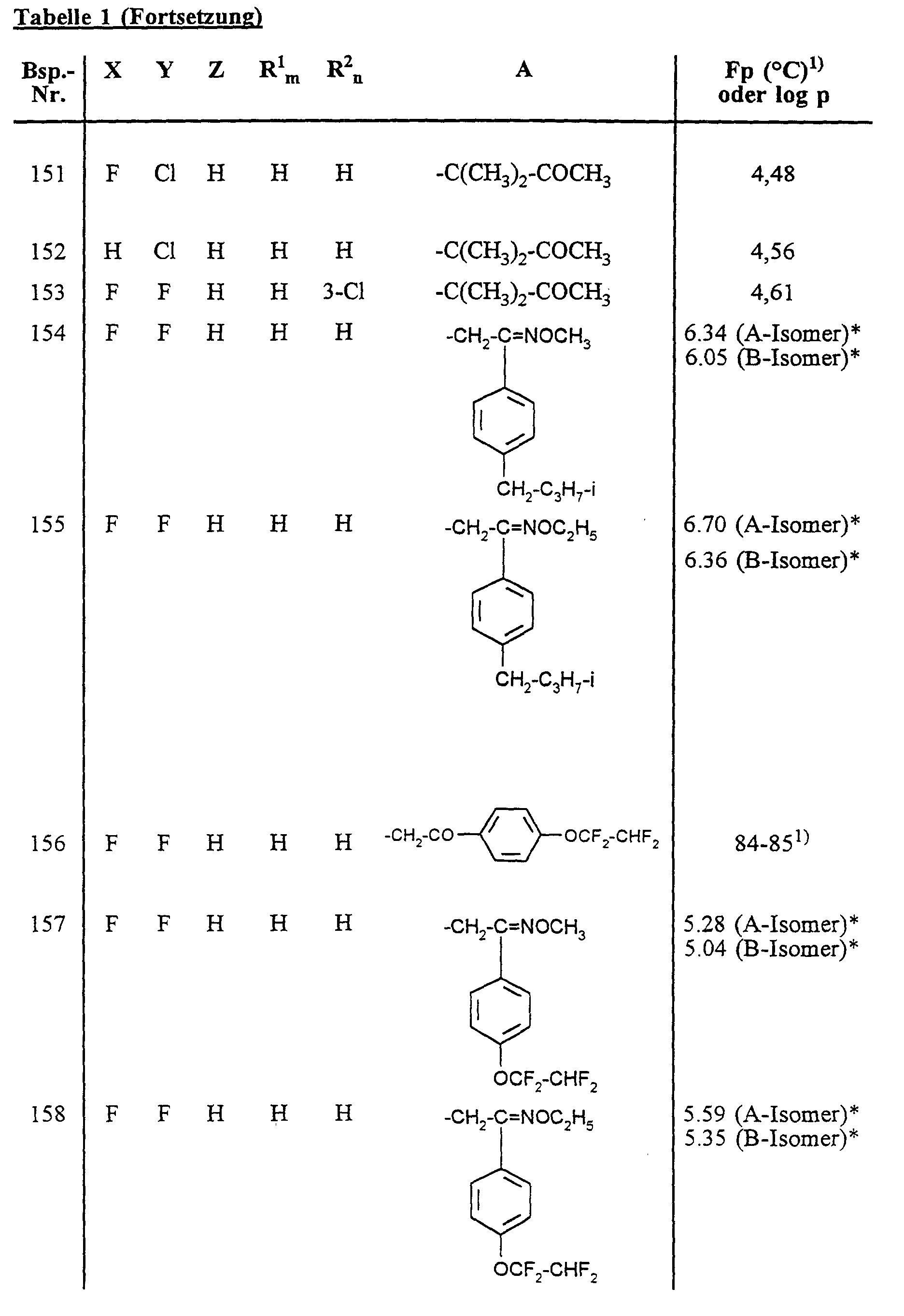

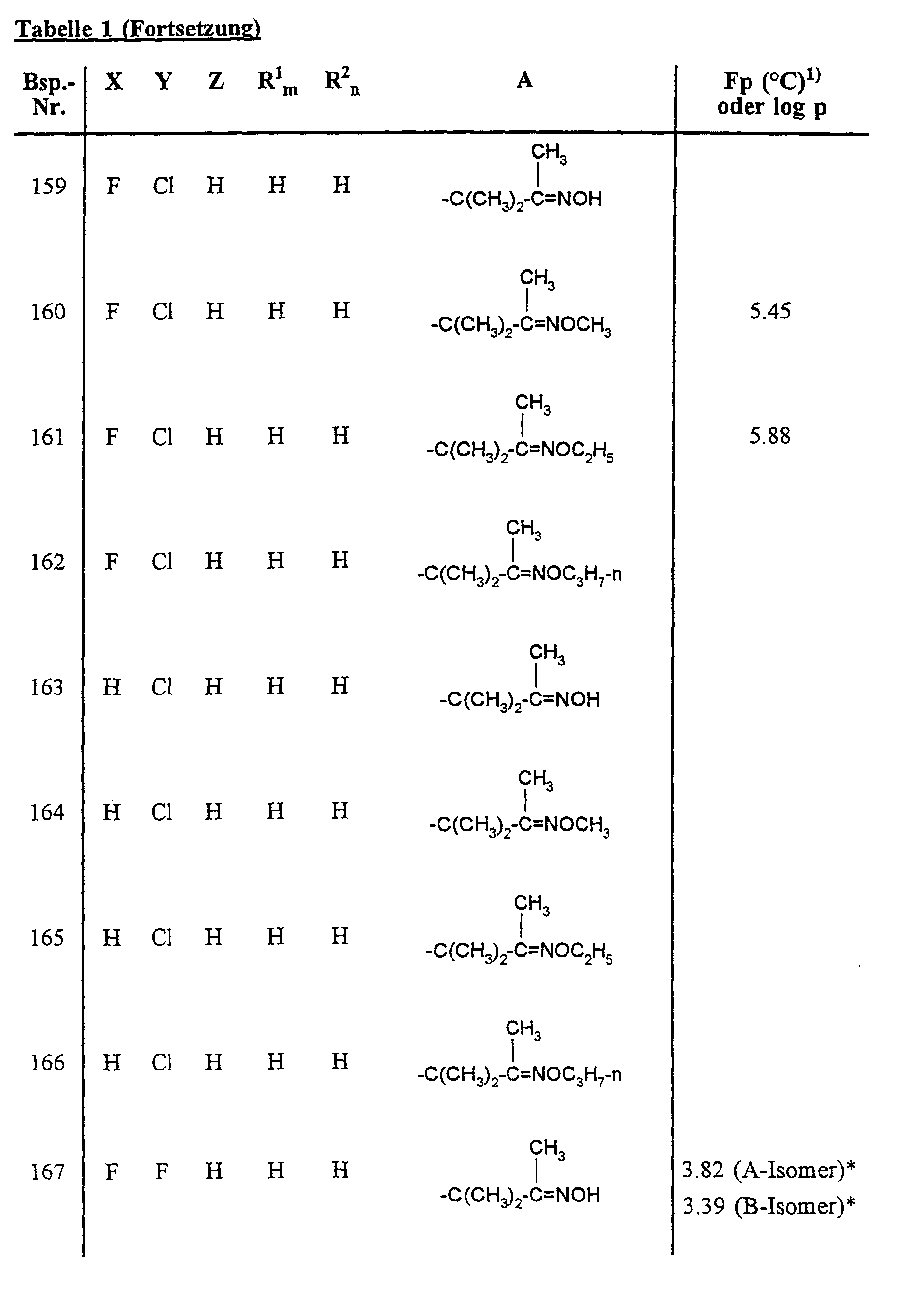

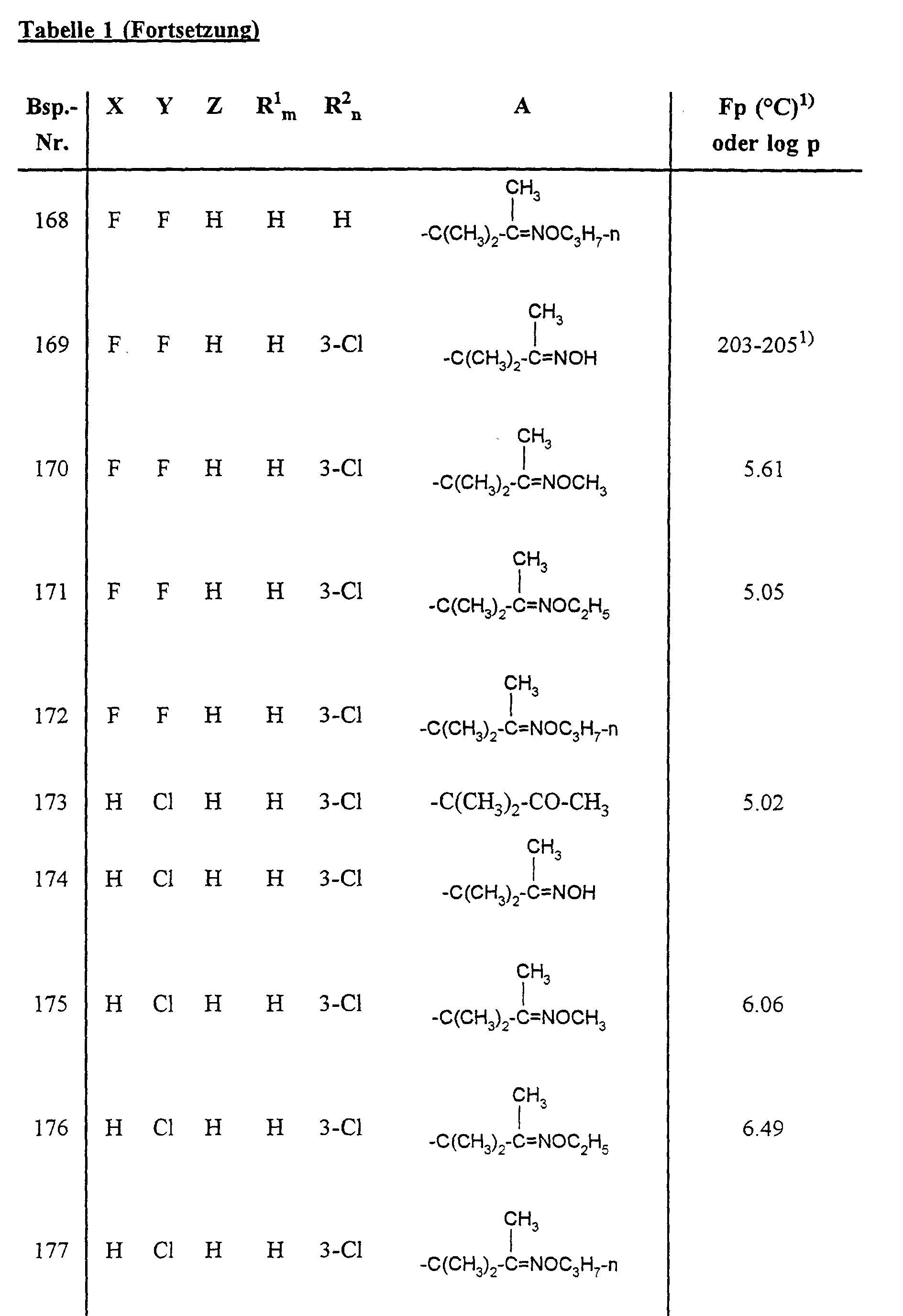

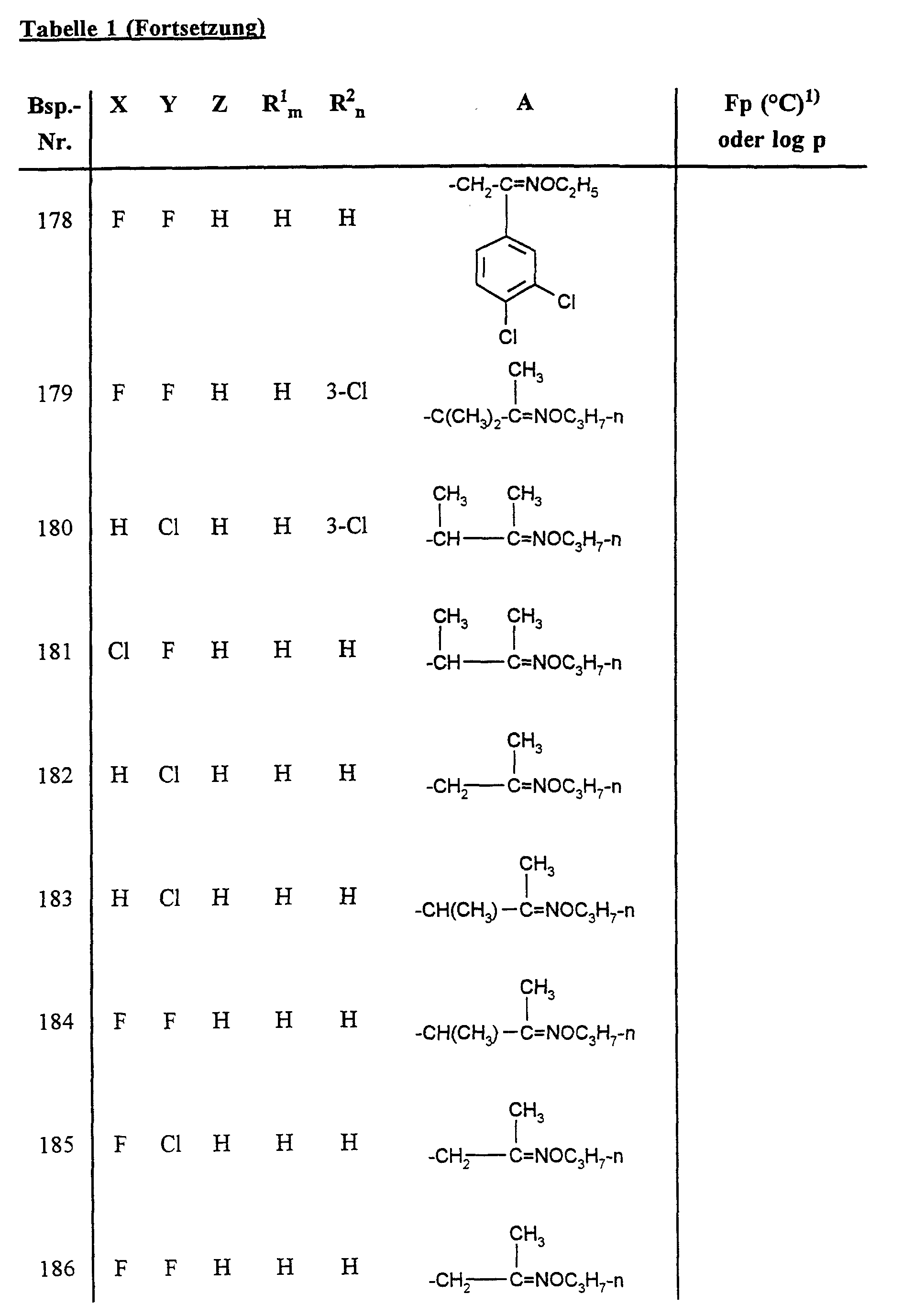

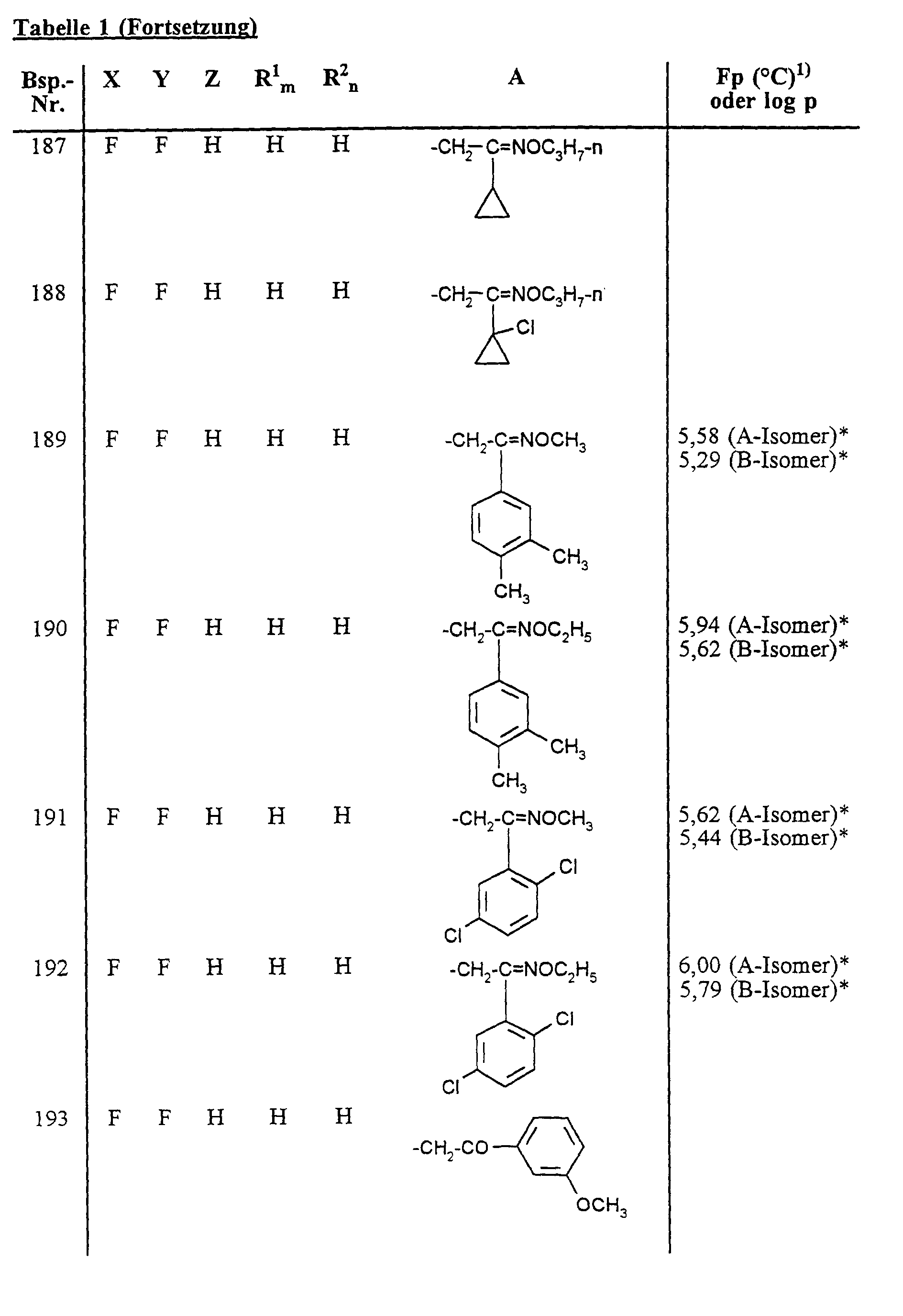

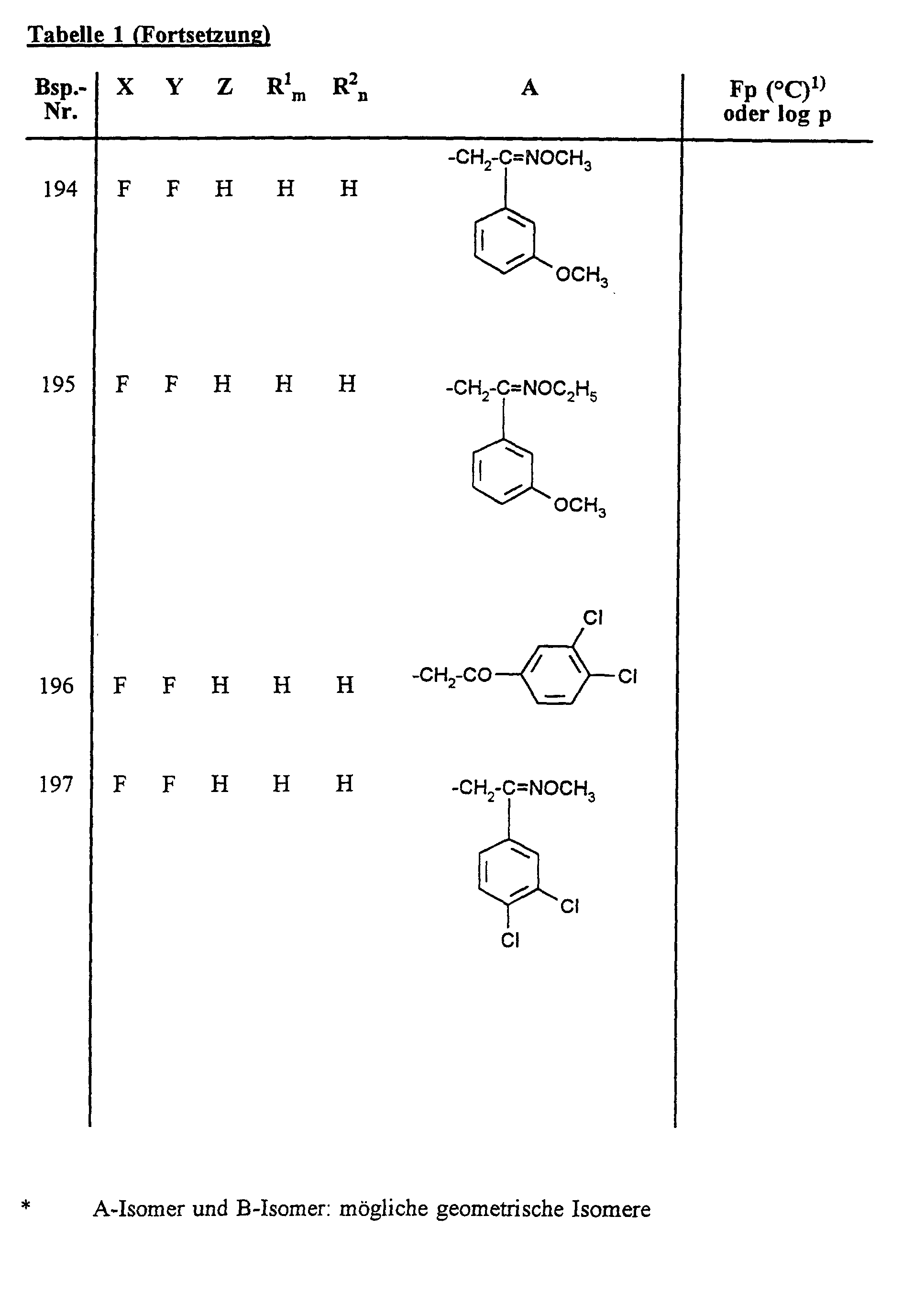

### Anwendungsbeispiele

In den folgenden Anwendungsbeispielen wurde die aus der EP-A 0 432 661 bekannte Verbindung der Formel (A) 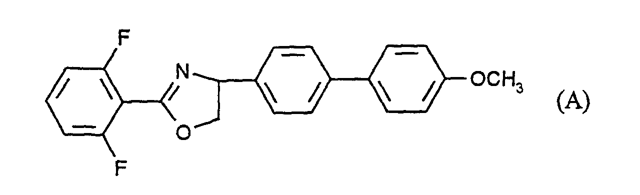 als Vergleichssubstanz eingesetzt.

### Beispiel A

### Phaedon-Larven-Test

- Lösungsmittel:: 7 Gewichtsteile Dimethylfomamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen der Herstellungsbeispiele 1, 2, 4, 13, 14, 16, 20 und 21 bei einer beispielhaften Wirkstoffkonzentration von 0,01 % eine Abtötung von 100 % nach 7 Tagen, während die bekannte Verbindung (A) keine Abtötung zeigte.

### Beispiel: B

### Plutella-Test

- Lösungsmittel:: 7 Gewichtsteile Dimethylfomamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen der Herstellungsbeispiele 2 und 4 bei einer beispielhaften Wirkstoffkonzentration von 0,0001 % eine Abtötung von 100 % nach 7 Tagen, während die bekannte Verbindung (A) eine Abtötung von lediglich 5 % bewirkte.

### Beispiel C

### Myzus-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Keimlinge der Dicken Bohne (Vicia faba), die von der Grünen Pfirsichblattlaus (Myzus persicae) befallen sind, werden in die Wirkstoffzubereitung der gewünschten Konzentration getaucht und in eine Plastikdose gelegt.

Nach der gewünschten Zeit wird die Abtötung in Prozent bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen der Herstellungsbeispiele 4, 17 und 21 bei einer beispielhaften Wirkstoffkonzentration von 0,01 % nach 6 Tagen eine Abtötung von 95 % bis 98 %, während die bekannte Verbindung (A) eine Abtötung von lediglich 5 % bewirkte.

### Beispiel D

### Spodoptera Frugiperda-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die in % bestimmt. Dabei bedeutet 100 %, daß alle Raupen abgetötet wurden; 0 % bedeutet, daß keine Raupen abgetötet wurden.

In diesem Test bewirkten z.B. die Verbindungen der Herstellungsbeispiele 1, 2, 4, 10, 14, 16, 18, 20 und 21 bei einer beispielhaften Wirkstoffkonzentration von 0,1 % nach 7 Tagen eine Abtötung von 100 %, während die bekannte Verbindung (A) keine Abtötung zeigte.

### Beispiel E

### Tetranychus-Test (OP-resistent/Tauchbehandlung)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

In diesem Test bewirkten z.B. die Verbindungen der Herstellungsbeispiele 1 und 2 bei einer beispielhaften Wirkstoffkonzentration von 0,001 % nach 13 Tagen eine Abtötung von 98 %, während die bekannte Verbindung (A) eine Abtötung von lediglich 45 % zeigte.

### Beispiel F

### Test mit Fliegenlarven / Entwicklungshemmende Wirkung

- Testtiere:: Alle larvalen Stadien von Lucilia cuprina (OP-resistent) [Puppen und Adulte (ohne Kontakt zum Wirkstoff)]
- Lösungsmittel:: 35 Gewichtsteile Ethylenglykolmonomethylether
- Emulgator:: 35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

30 - 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ± 10 %) aufbewahrt. Die Wirkungskontrolle erfolgt nach 24 Stunden und 48 Stunden (larvizide Wirkung). Nach dem Auswandern der Larven (ca. 72 Stunden) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 Stunden (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Flohentwicklung, bzw. ein Entwicklungsstillstand vor dem Adulten-Stadium. Dabei bedeutet 100 % larvizide Wirkung, daß nach 48 Stunden alle Larven abgestorben sind. 100 % entwicklungsinhibitorische Wirkung bedeutet, daß keine adulte Fliegen geschlüpft sind.

In diesem Test bewirkten z.B. die Verbindungen der Herstellungsbeispiele 4, 13, 15, 16, 17, 18, 20, 21, 23, 24, 25 und 26 bei einer beispielhaften Wirkstoffkonzentration von 1000 ppm eine Abtötung von 100 %.

## Patentansprüche

1. Verbindungen der Formel (I) 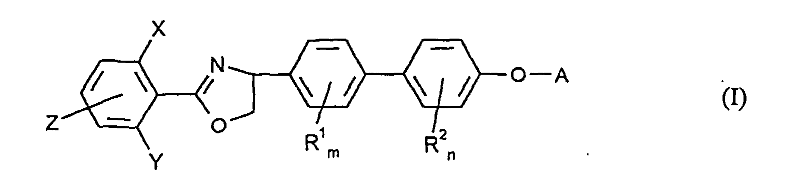 in welcher
X für Wasserstoff, Fluor oder Chlor steht,
Y für Fluor, Chlor oder Methyl steht,
Z für Wasserstoff, Fluor, Chlor, C₁-C₄-Alkyl, C₁-C₄-Alkoxy oder Di(C₁-C₄)alkylamino steht,
R¹ und R² unabhängig voneinander für Fluor, Chlor, C₁-C₄-Alkyl, C₁-C₄-Alkoxy, C₁-C₄-Alkylthio, C₁-C₄-Halogenalkyl, C₁-C₄-Halogenalkoxy oder C₁-C₄-Halogenalkylthio stehen,
m und n unabhängig voneinander für 0, 1 oder 2 stehen,
A für die Gruppierung (CH₂)ₚ-(CR³R⁴)_{q}-(CH₂)ᵣ-R steht,
wobei
R³ und R⁴ unabhängig voneinander für Wasserstoff oder C₁-C₄-Alkyl stehen,
p, q und r unabhängig voneinander für 0, 1, 2 oder 3 stehen, wobei mindestens ein Index ungleich 0 ist und die Summe der Indizes nicht größer als 5 ist,
R¹ und R² unabhängig voneinander für Fluor, Chlor, Methyl, Ethyl, Methoxy, Methylthio, Trifluormethyl, Trifluormethoxy oder Trifluormethylthio stehen,
m und n unabhängig voneinander für 0, 1 oder 2 stehen,
A für die Gruppierung (CH₂)ₚ-(CR³R⁴)_{q}-(CH₂)ᵣ-R, steht,
wobei
R³ und R⁴ unabhängig voneinander für Wasserstoff, Methyl, Ethyl, n- oder i-Propyl, n-, i-, s- oder t-Butyl stehen;
p, q und r unabhängig voneinander für 0, 1, 2 oder 3 stehen, wobei mindestens ein Index ungleich 0 ist,
R für Cyano; für einen der folgenden Heterocyclen 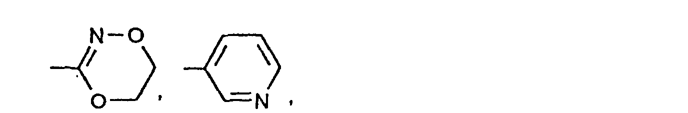 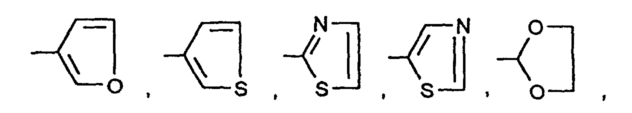 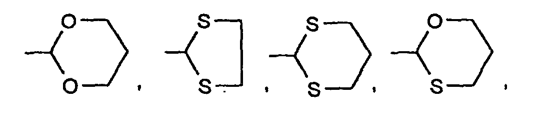 die gegebenenfalls einfach bis dreifach, gleich oder verschieden substituiert sind durch Fluor, Chlor, Brom, Methyl, Ethyl, n- oder i-Propyl oder Trifluormethyl;
oder für eine der folgenden Gruppierungen:
(a) ―CO-R⁵
(b) ―CO-OR⁶
(c) ―CO-NR⁷R⁸
(d) ―CS-NR⁷R⁸
(e) 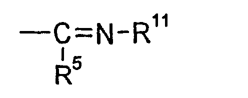
(f) 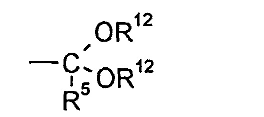
(g) 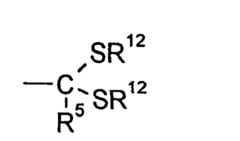
(h) 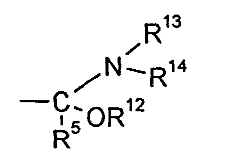
(i) 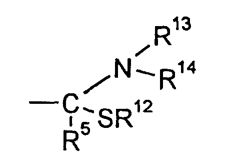
(j) 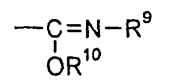
(k) 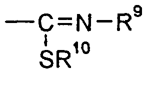 steht,
wobei
R⁵ für Wasserstoff; Methyl, Ethyl, n- oder i-Propyl, die isomeren Butyle, die isomeren Pentyle, die isomeren Hexyle; C₁-C₂-Halogenalkyl mit 1 bis 5 gleichen oder verschiedenen Halogenatomen, wie F- und Cl-Atomen, C₃-C₆-Alkenyl, C₃-C₆-Halogenalkenyl mit 1 bis 5 gleichen oder verschiedenen Halogenatomen, wie F- und Cl-Atomen; jeweils gegebenenfalls einfach bis dreifach, gleich oder verschieden durch Fluor, Chlor, Methyl, Ethyl, n- oder i-Propyl oder C₁-C₂-Halogenalkyl mit 1 bis 5 gleichen oder verschiedenen Halogenatomen, wie F- und Cl-Atomen substituiertes Cyclopropyl, Cyclopentyl oder Cyclohexyl,
oder für gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Methylcarbonylamino, Ethylcarbonylamino, Methylcarbonyl-methylamino, Cyano, Nitro, -COOR¹⁵ oder -CONR¹⁶R¹⁷ substituiertes Phenyl steht,
R⁶ für Wasserstoff, Methyl, Ethyl, n- oder i-Propyl, n-, i-, s-oder t-Butyl; C₁-C₂-Halogenalkyl mit 1 bis 5 gleichen oder verschiedenen Halogenatomen, wie F- und Cl-Atomen; gegebenenfalls einfach oder mehrfach durch Fluor und/oder Chlor substituiertes Allyl; jeweils gegebenenfalls einfach bis dreifach, gleich oder verschieden durch Fluor, Chlor, Methyl, Ethyl, n- oder i-Propyl oder C₁-C₂-Halogenalkyl mit 1 bis 5 gleichen oder verschiedenen Halogenatomen, wie F- und Cl-Atomen substituiertes Cyclopropyl, Cyclopentyl, Cyclohexyl, Cyclopropyl-C₁-C₂-alkyl, Cyclopentyl-C₁-C₂-alkyl oder Cyclohexyl-C₁-C₂-alkyl,
oder für jeweils gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, -COOR¹⁵ oder -CONR¹⁶R¹⁷ substituiertes Phenyl-C₁-C₂-alkyl oder Naphthyl-C₁-C₂-alkyl steht,
R⁷ und R⁸ unabhängig voneinander für Wasserstoff, C₁-C₄-Alkyl, Methoxy, Ethoxy, C₁-C₃-Halogenalkyl mit 1 bis 5 gleichen oder verschiedenen Halogenatomen, wie F- und Cl-Atomen; gegebenenfalls einfach oder mehrfach durch Fluor und/oder Chlor substituiertes Allyl; jeweils gegebenenfalls einfach bis dreifach, gleich oder verschieden durch Fluor, Chlor, Methyl, Ethyl, n- oder i-Propyl oder Trifluormethyl substituiertes Cyclopropyl, Cyclopentyl, Cyclohexyl, Cyclopropylmethyl, Cyclopentylmethyl oder Cyclohexylmethyl,
oder für jeweils gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, -COOR¹⁵ oder -CONR¹⁶R¹⁷ substituiertes Phenyl oder Phenyl-C₁-C₂-alkyl,
oder für -OR⁶ oder -NR⁵R⁶ stehen,
R⁹ und R¹⁰ unabhängig voneinander für Methyl, Ethyl, n- oder i-Propyl stehen;
R¹¹ für -OR⁶, -NR⁵R⁶ oder -N(R⁵)-COOR⁶ steht,
und
R¹², R¹³ und R¹⁴ unabhängig voneinander für Methyl, Ethyl, n-oder i-Propyl stehen,
R¹⁵ für Wasserstoff, Methyl, Ethyl, n- oder i-Propyl; C₁-C₂-Halogenalkyl mit 1 bis 5 gleichen oder verschiedenen Halogenatomen, wie F- und Cl-Atomen; jeweils gegebenenfalls einfach bis dreifach, gleich oder verschieden durch Fluor, Chlor, Methyl, Ethyl, n- oder i-Propyl oder C₁-C₂-Halogenalkyl mit 1 bis 5 gleichen oder verschiedenen Halogenatomen, wie F- und Cl-Atomen substituiertes Cyclopropyl, Cyclopentyl oder Cyclohexyl,
oder für gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, C₁-C₄-Alkoxycarbonyl oder Di(C₁-C₄)alkylaminocarbonyl substituiertes Phenyl steht,
R¹⁶ und R¹⁷ unabhängig voneinander für Wasserstoff, C₁-C₄-Alkyl, Methoxy, Ethoxy, C₁-C₃-Halogenalkyl mit 1 bis 5 gleichen oder verschiedenen Halogenatomen, wie F- und Cl-Atomen; gegebenenfalls einfach oder mehrfach durch Fluor und/oder Chlor substituiertes Allyl; jeweils gegebenenfalls einfach bis dreifach, gleich oder verschieden durch Fluor, Chlor, Methyl, Ethyl, n- oder i-Propyl oder Trifluormethyl substituiertes Cyclopropyl, Cyclopentyl, Cyclohexyl, Cyclopropylmethyl, Cyclopentylmethyl oder Cyclohexylmethyl, oder für jeweils gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, C₁-C₄-Alkoxycarbonyl oder Di(C₁-C₄)alkylaminocarbonyl substituiertes Phenyl oder Phenyl-C₁-C₂-alkyl
oder für -OR⁶ oder -NR⁵R⁶ stehen.

2. Verbindungen der Formel (I) gemäß Anspruch 1, in welcher
X für Wasserstoff, Fluor oder Chlor steht,
Y für Fluor oder Chlor steht,
Z für Wasserstoff, Fluor, Chlor, Methyl, Ethyl, Methoxy, Dimethylamino oder Diethylamino steht,
R¹ und R² unabhängig voneinander für Fluor, Chlor, Methyl, Ethyl, Methoxy, Methylthio, Trifluormethyl, Trifluormethoxy oder Trifluormethylthio stehen,
m und n unabhängig voneinander für 0, 1 oder 2 stehen,
A für die Gruppierung (CH₂)ₚ-(CR³R⁴)_{q}-(CH₂)ᵣ-R, steht,
wobei
R³ und R⁴ unabhängig voneinander für Wasserstoff, Methyl, Ethyl, n- oder i-Propyl, n-, i-, s- oder t-Butyl stehen;
p, q und r unabhängig voneinander für 0, 1, 2 oder 3 stehen, wobei mindestens ein Index ungleich 0 ist,
R für Cyano; für einen der folgenden Heterocyclen 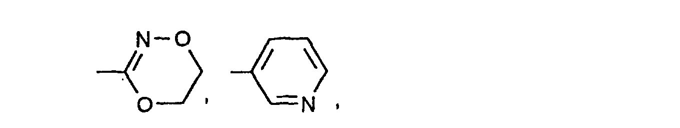 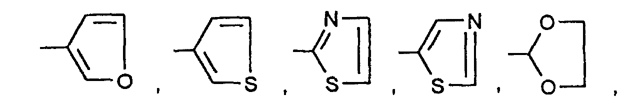 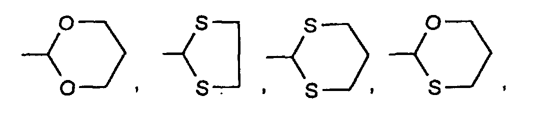 die gegebenenfalls einfach bis dreifach, gleich oder verschieden substituiert sind durch Fluor, Chlor, Brom, Methyl, Ethyl, n- oder i-Propyl oder Trifluormethyl;
oder für eine der folgenden Gruppierungen:
(a) ―CO-R⁵
(b) ―CO-OR⁶
(c) ―CO-NR⁷R⁸
(d) ―CS-NR⁷R⁸
(e) 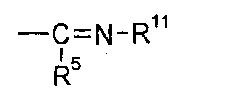
(f) 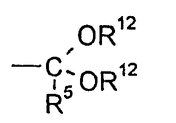
(g) 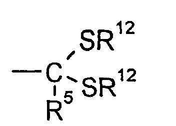
(h) 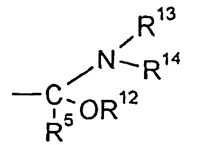
(i) 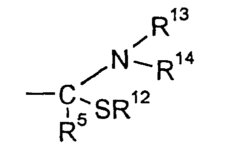
(j) 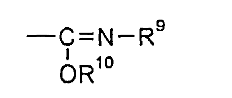
(k) 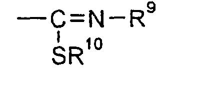 steht,
wobei
R⁵ für Wasserstoff; Methyl, Ethyl, n- oder i-Propyl, die isomeren Butyle, die isomeren Pentyle, die isomeren Hexyle; C₁-C₂-Halogenalkyl mit 1 bis 5 gleichen oder verschiedenen Halogenatomen, wie F- und Cl-Atomen, C₃-C₆-Alkenyl, C₃-C₆-Halogenalkenyl mit 1 bis 5 gleichen oder verschiedenen Halogenatomen, wie F- und Cl-Atomen; jeweils gegebenenfalls einfach bis dreifach, gleich oder verschieden durch Fluor, Chlor, Methyl, Ethyl, n- oder i-Propyl oder C₁-C₂-Halogenalkyl mit 1 bis 5 gleichen oder verschiedenen Halogenatomen, wie F- und Cl-Atomen substituiertes Cyclopropyl, Cyclopentyl oder Cyclohexyl,
oder für gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Methylcarbonylamino, Ethylcarbonylamino, Methylcarbonyl-methylamino, Cyano, Nitro, -COOR¹⁵ oder -CONR¹⁶R¹⁷ substituiertes Phenyl steht,
R⁶ für Wasserstoff, Methyl, Ethyl, n- oder i-Propyl, n-, i-, s-oder t-Butyl; C₁-C₂-Halogenalkyl mit 1 bis 5 gleichen oder verschiedenen Halogenatomen, wie F- und Cl-Atomen; gegebenenfalls einfach oder mehrfach durch Fluor und/oder Chlor substituiertes Allyl; jeweils gegebenenfalls einfach bis dreifach, gleich oder verschieden durch Fluor, Chlor, Methyl, Ethyl, n- oder i-Propyl oder C₁-C₂-Halogenalkyl mit 1 bis 5 gleichen oder verschiedenen Halogenatomen, wie F- und Cl-Atomen substituiertes Cyclopropyl, Cyclopentyl, Cyclohexyl, Cyclopropyl-C₁-C₂-alkyl, Cyclopentyl-C₁-C₂-alkyl oder Cyclohexyl-C₁-C₂-alkyl,
oder für jeweils gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, -COOR¹⁵ oder -CONR¹⁶R¹⁷ substituiertes Phenyl-C₁-C₂-alkyl oder Naphthyl-C₁-C₂-alkyl steht,
R⁷ und R⁸ unabhängig voneinander für Wasserstoff, C₁-C₄-Alkyl, Methoxy, Ethoxy, C₁-C₃-Halogenalkyl mit 1 bis 5 gleichen oder verschiedenen Halogenatomen, wie F- und Cl-Atomen; gegebenenfalls einfach oder mehrfach durch Fluor und/oder Chlor substituiertes Allyl; jeweils gegebenenfalls einfach bis dreifach, gleich oder verschieden durch Fluor, Chlor, Methyl, Ethyl, n- oder i-Propyl oder Trifluormethyl substituiertes Cyclopropyl, Cyclopentyl, Cyclohexyl, Cyclopropylmethyl, Cyclopentylmethyl oder Cyclohexylmethyl,
oder für jeweils gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, -COOR¹⁵ oder -CONR¹⁶R¹⁷ substituiertes Phenyl oder Phenyt-C₁-C₂-alkyl,
oder für -OR⁶ oder -NR⁵R⁶ stehen,
R⁹ und R¹⁰ unabhängig voneinander für Methyl, Ethyl, n- oder i-Propyl stehen;
R¹¹ für -OR⁶, -NR⁵R⁶ oder -N(R⁵)-COOR⁶ steht,
und
R¹², R¹³ und R¹⁴ unabhängig voneinander für Methyl, Ethyl, n-oder i-Propyl stehen,
R¹⁵ für Wasserstoff, Methyl, Ethyl, n- oder i-Propyl; C₁-C₂-Halogenalkyl mit 1 bis 5 gleichen oder verschiedenen Halogenatomen, wie F- und Cl-Atomen; jeweils gegebenenfalls einfach bis dreifach, gleich oder verschieden durch Fluor, Chlor, Methyl, Ethyl, n- oder i-Propyl oder C₁-C₂-Halogenalkyl mit 1 bis 5 gleichen oder verschiedenen Halogenatomen, wie F- und Cl-Atomen substituiertes Cyclopropyl, Cyclopentyl oder Cyclohexyl,
oder für gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, C₁-C₄-Alkoxycarbonyl oder Di(C₁-C₄)alkylaminocarbonyl substituiertes Phenyl steht,
R¹⁶ und R¹⁷ unabhängig voneinander für Wasserstoff, C₁-C₄-Alkyl, Methoxy, Ethoxy, C₁-C₃-Halogenalkyl mit 1 bis 5 gleichen oder verschiedenen Halogenatomen, wie F- und Cl-Atomen; gegebenenfalls einfach oder mehrfach durch Fluor und/oder Chlor substituiertes Allyl; jeweils gegebenenfalls einfach bis dreifach, gleich oder verschieden durch Fluor, Chlor, Methyl, Ethyl, n- oder i-Propyl oder Trifluormethyl substituiertes Cyclopropyl, Cyclopentyl, Cyclohexyl, Cyclopropylmethyl, Cyclopentylmethyl oder Cyclohexylmethyl, oder für jeweils gegebenenfalls einfach oder zweifach, gleich oder verschieden durch Fluor, Chlor, Methyl, Ethyl, Methoxy, Ethoxy, Methylthio, Trifluormethyl, Trifluormethoxy, Trifluormethylthio, Dimethylamino, Diethylamino, Cyano, Nitro, C₁-C₄-Alkoxycarbonyl oder Di(C₁-C₄)alkylaminocarbonyl substituiertes Phenyl oder Phenyl-C₁-C₂-alkyl
oder für -OR⁶ oder -NR⁵R⁶ stehen.

3. Verbindungen der Formel (X) 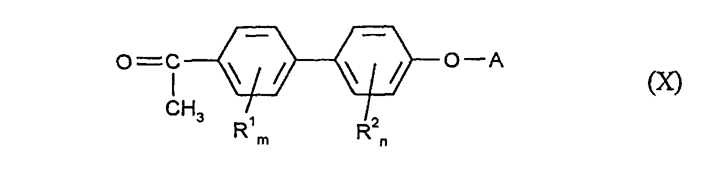 in welcher
A, R¹, R², m und n die in Anspruch 1 angegebene Bedeutung haben.

4. Verbindungen der Formel (XI) 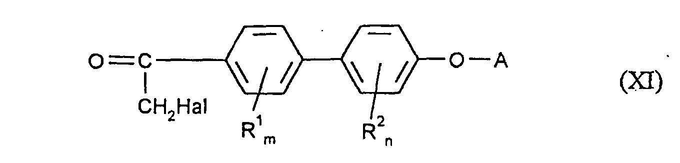 in welcher
A, R¹, R², m und n die in Anspruch 1 angegebene Bedeutung haben und
Hal für Chlor oder Brom steht.

5. Verbindungen der Formel (XII) 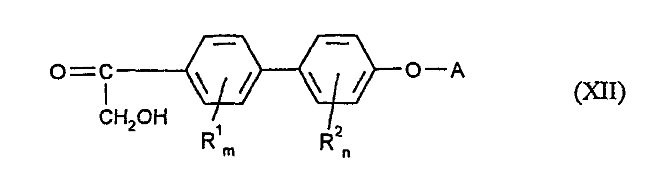 in welcher
A, R¹, R², m und n die in Anspruch 1 angegebene Bedeutung haben.

6. Verbindungen der Formel (XIII) 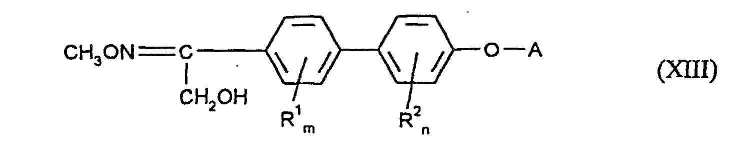 in welcher
A, R¹, R², m und n die in Anspruch 1 angegebene Bedeutung haben.

7. Verbindungen der Formel (XIV) 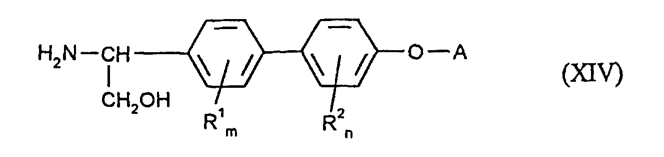 in welcher
A, R¹, R², m und n die in Anspruch 1 angegebene Bedeutung haben.

8. Verbindungen der Formel (XVI) 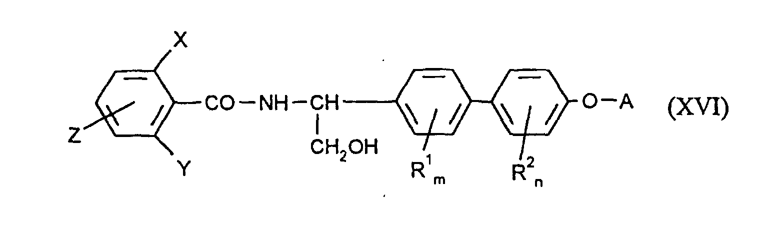 in welcher
A, R¹, R², m, n, X, Y und Z die in Anspruch 1 angegebene Bedeutung haben.

9. Verbindungen der Formel (XVII) 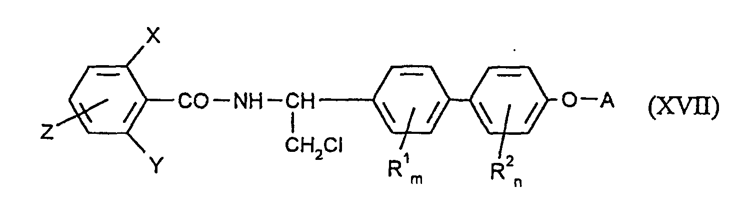 in welcher
A, R¹, R², m, n, X, Y und Z die in Anspruch 1 angegebene Bedeutung haben.

10. Verfahren zur Herstellung von Verbindungen der Formel (I) gemäß Anspruch 1, **dadurch gekennzeichnet, daß** man Hydroxibiphenyloxazoline der Formel (II) 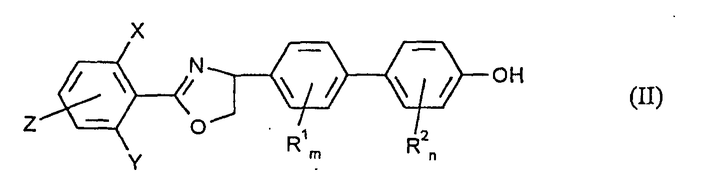 in welcher
X, Y, Z, R¹, R², m und n die in Anspruch 1 angegebene Bedeutung haben,
mit einer Verbindung der Formel (III)
M-A (III)
in welcher
A die in Anspruch 1 angegebene Bedeutung hat und
M für eine Abgangsgruppe steht,
gegebenenfalls in Gegenwart einer Base und/oder eines Katalysators sowie gegebenenfalls in Gegenwart eines Verdünnungsmittels umsetzt.

11. Schädlingsbekämpfungsmittel, **gekennzeichnet durch** einen Gehalt an mindestens einer Verbindung der Formel (I) gemäß Anspruch 1.

12. Verwendung von Verbindungen der Formel (I) gemäß Anspruch 1 zur Bekämpfung von Schädlingen.

13. Verfahren zur Bekämpfung von Schädlingen, **dadurch gekennzeichnet, daß** man Verbindungen der Formel (I) gemäß Anspruch 1 auf Schädlinge und/oder ihren Lebensraum einwirken läßt.

14. Verfahren zur Herstellung von Schädlingsbekämpfungsmitteln, **dadurch gekennzeichnet, daß** man Verbindungen der Formel (I) gemäß Anspruch 1 mit Streckmitteln und/oder oberflächenaktiven Mitteln vermischt.

15. Verwendung von Verbindungen der Formel (I) gemäß Anspruch 1 zur Herstellung von Schädlingsbekämpfungsmitteln.

## Claims

1. Compounds of the formula (I) 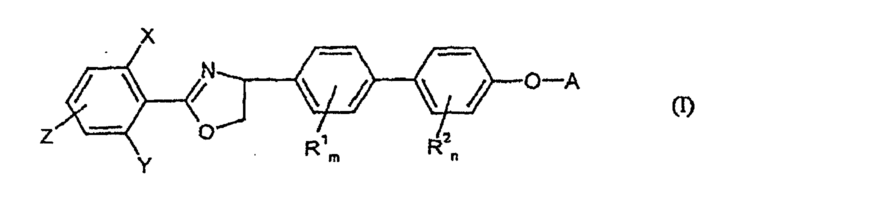 in which
X represents hydrogen, fluorine or chlorine,
Y represents fluorine, chlorine or methyl,
Z represents hydrogen, fluorine, chlorine, C₁-C₄-alkyl, C₁-C₄-alkoxy or di(C₁-C₄)alkylamino,
R¹ and R² independently of one another represent fluorine, chlorine, C₁-C₄-alkyl, C₁-C₄-alkoxy, C₁-C₄-alkylthio, C₁-C₄-halogenoalkyl, C₁-C₄-halogenoalkoxy or C₁-C₄-halogenoalkylthio,
m and n independently of one another represent 0, 1 or 2,
A represents the grouping (CH₂)ₚ-(CR³R⁴)_{q}-(CH₂)ᵣ-R, wherein
R³ and R⁴ independently of one another represent hydrogen or C₁-C₄-alkyl,
p, q and r independently of one another represent 0, 1, 2 or 3, at least one index being other than 0 and the sum of the indices being not greater than 5,
R represents cyano; or represents one of the heterocycles below 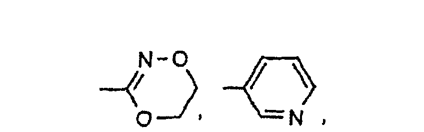 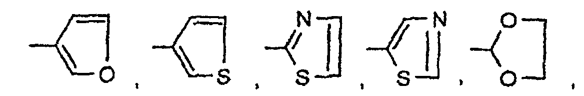 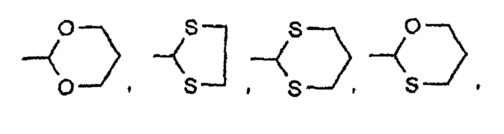
which are optionally mono- to trisubstituted by identical or different substituents from the group consisting of fluorine, chlorine, bromine, methyl, ethyl, n- or isopropyl and trifluoromethyl;
or represents one of the following groupings:
(a) ―CO-R⁵
(b) ―CO-OR⁶
(c) ―CO-NR⁷R⁸
(d) ―CS-NR⁷R⁸
(e) 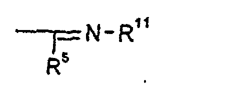
(f) 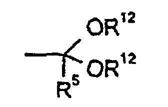
(g) 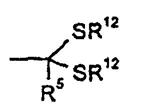
(h) 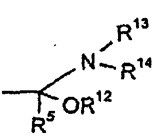
(i) 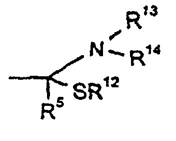
(j) 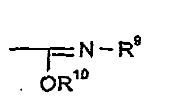
(k) 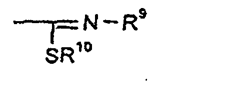
wherein
R⁵ represents hydrogen, methyl, ethyl, nor isopropyl, the isomeric butyls, the isomeric pentyls, the isomeric hexyls; C₁-C₂-halogenoalkyl having 1 to 5 identical or different halogen atoms, such as F and Cl atoms, C₃-C₆-alkenyl, C₃-C₆-haloalkenyl having 1 to 5 identical or different halogen atoms, such as F and Cl atoms; cyclopropyl, cyclopentyl or cyclohexyl, each of which is optionally mono- to trisubstituted by identical or different substituents from the group consisting of fluorine, chlorine, methyl, ethyl, n- or isopropyl and C₁-C₂-halogenoalkyl having 1 to 5 identical or different halogen atoms, such as F and Cl atoms,
or represents phenyl which is optionally mono- or disubstituted by identical or different substituents from the group consisting of fluorine, chlorine, methyl, ethyl, methoxy, ethoxy, methylthio, trifluoromethyl, trifluoromethoxy, trifluoromethylthio, dimethylamino, diethylamino, methylcarbonylamino, ethylcarbonylamino, methylcarbonylmethylamino, cyano, nitro, -COOR¹⁵ and -CONR¹⁶R¹⁷,
R⁶ represents hydrogen, methyl, ethyl, nor isopropyl, n-, iso-, s- or t-butyl; C₁-C₂-halogenoalkyl having 1 to 5 identical or different halogen atoms, such as F and Cl atoms; allyl which is optionally mono- or polysubstituted by fluorine and/or chlorine; cyclopropyl, cyclopentyl, cyclohexyl, cyclopropyl-C₁-C₂-alkyl, cyclopentyl-C₁-C₂-alkyl or cyclohexyl-C₁-C₂-alkyl, each of which is optionally mono- to trisubstituted by identical or different substituents from the group consisting of fluorine, chlorine, methyl, ethyl, n- or isopropyl and C₁-C₂-halogenoalkyl having 1 to 5 identical or different halogen atoms, such as F and Cl atoms,
or represents phenyl-C₁-C₂-alkyl or naphthyl C₁-C₂-alkyl, each of which is optionally mono- or disubstituted by identical or different substituents from the group consisting of fluorine, chlorine, methyl, ethyl, methoxy, ethoxy, methylthio, trifluoromethyl, trifluoromethoxy, trifluoromethylthio, dimethylamino, diethylamino, cyano, nitro, -COOR¹⁵ and -CONR¹⁶R¹⁷,
R⁷ and R⁸ independently of one another represent hydrogen, C₁-C₄-alkyl, methoxy, ethoxy, C₁-C₃-halogenoalkyl having 1 to 5 identical or different halogen atoms, such as F and Cl atoms; allyl which is optionally mono- or polysubsituted by fluorine and/or chlorine; cyclopropyl, cyclopentyl, cyclohexyl, cyclopropylmethyl, cyclopentylmethyl or cyclohexylmethyl, each of which is optionally mono- to trisubstituted by identical or different substituents from the group consisting of fluorine, chlorine, methyl, ethyl, n- or isopropyl and trifluoromethyl,
or represent phenyl or phenyl-C₁-C₂-alkyl, each of which is optionally mono- or disubstituted by identical or different substituents from the group consisting of fluorine, chlorine, methyl, ethyl, methoxy, ethoxy, methylthio, trifluoromethyl, trifluoromethoxy, trifluoromethylthio, dimethylamino, diethylamino, cycano, nitro, -COOR¹⁵ and -CONR¹⁶R¹⁷,
or represent -OR⁶ or -NR⁵R⁶,
R⁹ and R¹⁰ independently of one another represent methyl, ethyl, n- or isopropyl;
R¹¹ represents -OR⁶, -NR⁵R⁶ or -N(R⁵)-COOR⁶,
and
R¹², R¹³ and R¹⁴ independently of one another represent methyl, ethyl, n- or isopropyl,
R¹⁵ represents hydrogen, methyl, ethyl, n- or isopropyl; C₁-C₂-halogenoalkyl having 1 to 5 identical or different halogen atoms, such as F and Cl atoms; cyclopropyl, cyclopentyl or cyclohexyl, each of which is optionally mono- to trisubstituted by identical or different substituents from the group consisting of fluorine, chlorine, methyl, ethyl, n- or isopropyl and C₁-C₂-halogenoalkyl having 1 to 5 identical or different halogen atoms, such as F and Cl atoms, or represents phenyl which is optionally mono- or disubstituted by identical or different substituents from the group consisting of fluorine, chlorine, methyl, ethyl, methoxy, ethoxy, methylthio, trifluoromethyl, trifluoromethoxy, trifluoromethylthio, dimethylamino, diethylamino, cyano, nitro, C₁-C₄-alkoxycarbonyl and di (C₁-C₄) akylaminocarbonyl,
R¹⁶ and R¹⁷ independently of one another represent hydrogen, C₁-C₄-alkyl, methoxy, ethoxy, C₁-C₃-halogenoalkyl having 1 to 5 identical or different halogen atoms, such as F and Cl atoms; allyl which is optionally mono- or polysubsituted by fluorine and/or chlorine; cyclopropyl, cyclopentyl, cyclohexyl, cyclopropylmethyl, cyclopentylmethyl or cyclohexylmethyl, each of which is optionally mono- to trisubstituted by identical or different substituents from the group consisting of fluorine, chlorine, methyl, ethyl, n- or isopropyl and trifluoromethyl, or represent phenyl or phenyl-C₁-C₂-alkyl, each of which is optionally mono- or disubstituted by identical or different substituents from the group consisting of fluorine, chlorine, methyl, ethyl, methoxy, ethoxy, methylthio, trifluoromethyl, trifluoromethoxy, trifluoromethylthio, dimethylamino, diethylamino, cyano, nitro, C₁-C₄-alkoxycarbonyl and di(C₁-C₄)alkylaminocarbonyl
or represent -OR⁶ or -NR⁵R⁶.

2. Compounds of the formula (I) according to Claim 1, in which
X represents hydrogen, fluorine or chlorine,
Y represents fluorine, chlorine or methyl,
Z represents hydrogen, fluorine, chlorine, C₁-C₄-alkyl, C₁-C₄-alkoxy or di(C₁-C₄)alkylamino,
R¹ and R² independently of one another represent fluorine, chlorine, C₁-C₄-alkyl, C₁-C₄-alkoxy, C₁-C₄-alkylthio, C₁-C₄-halogenoalkyl, C₁-C₄-halogenoalkoxy or C₁-C₄-halogenoalkylthio,
m and n independently of one another represent 0, 1 or 2,
A represents the grouping (CH₂)ₚ-(CR³R⁴)_{q}-(CH₂)ᵣ-R,
wherein
R³ and R⁴ independently of one another represent hydrogen or C₁-C₄-alkyl,
p, q and r independently of one another represent 0, 1, 2 or 3, at least one index being other than 0 and the sum of the indices being not greater than 5,
R represents cyano; or represents one of the following heterocyclic radicals 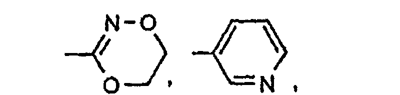 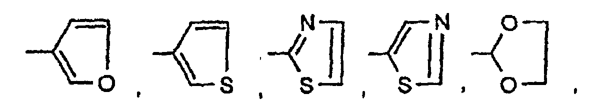 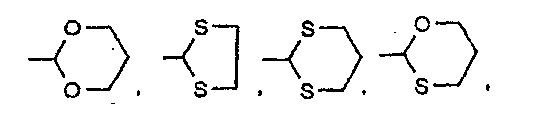
which are optionally substituted once to three times in an identical or different manner by fluorine, chlorine, bromine, methyl, ethyl, nor isopropyl or trifluoromethyl;
or represents one of the following groupings:
(a) ―CO-R⁵
(b) ―CO-OR⁶
(c) ―CO-N⁷R⁸
(d) ―CS-NR⁷R⁸
(e) 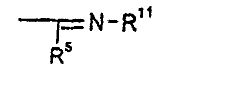
(f) 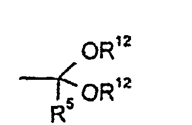
(g) 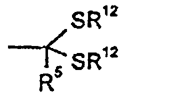
(h) 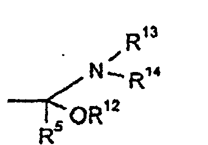
(i) 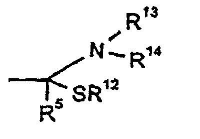
(j) 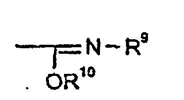
(k) 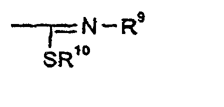
wherein
R⁵ represents hydrogen; methyl, ethyl, n- or isopropyl, the isomeric butyls, the isomeric pentyls or the isomeric hexyls; or C₁-C₂-halogenoalkyl having 1 to 5 identical or different halogen atoms, such as F and Cl atoms, C₃-C₆-alkenyl or C₃-C₆-halogenoalkenyl having 1 to 5 identical or different halogen atoms, such as F and Cl atoms; or cyclopropyl, cyclopentyl or cyclohexyl, in each case optionally substituted once to three times in an identical or different manner by fluorine, chlorine, methyl, ethyl, n- or isopropyl or C₁-C₂-halogenoalkyl having 1 to 5 identical or different halogen atoms, such as F and Cl atoms,
or represents phenyl which is optionally substituted once or twice in an identical or different manner by fluorine, chlorine, methyl, ethyl, methoxy, ethoxy, methylthio, trifluoromethyl, trifluoromethoxy, trifluoromethylthio, dimethylamino, diethylamino, methylcarbonylamino, ethylcarbonylamino, methylcarbonyl-methylamino, cyano, nitro, -COOR¹⁵ or -CONR¹⁶R¹⁷,
R⁶ represents hydrogen, methyl, ethyl, n- or isopropyl or n-, i-, s- or t-butyl; or C₁-C₂-halogenoalkyl having 1 to 5 identical or different halogen atoms, such as F and Cl atoms; or allyl which is optionally substituted once or several times by fluorine and/or chlorine; or cyclopropyl, cyclopentyl, cyclohexyl, cyclopropyl-C₁-C₂-alkyl, cyclopentyl-C₁-C₂-alkyl or cyclohexyl-C₁-C₂-alkyl, in each case optionally substituted once to three times in an identical or different manner by fluorine, chlorine, methyl, ethyl, n- or isopropyl or C₁-C₂-halogenoalkyl having 1 to 5 identical or different halogen atoms, such as F and Cl atoms,
or represents phenyl-C₁-C₂-alkyl or naphthyl-C₁-C₂-alkyl, in each case optionally substituted once or twice in an identical or different manner by fluorine, chlorine, methyl, ethyl, methoxy, ethoxy, methylthio, trifluoromethyl, trifluorodiethylamino, cyano, nitro, -COOR¹⁵ or -CONR¹⁶R¹⁷,
R⁷ and R⁸ independently of one another represent hydrogen, C₁-C₄-alkyl, methoxy, ethoxy or C₁-C₃-halogenoalkyl having 1 to 5 identical or different halogen atoms, such as F and Cl atoms; or allyl which is optionally substituted once or several times by fluorine and/or chlorine; or cyclopropyl, cyclopentyl, cyclohexyl, cyclopropylmethyl, cyclopentylmethyl or cyclohexylmethyl, in each case optionally substituted once to three times in an identical or different manner by fluorine, chlorine, methyl, ethyl, n- or isopropyl or trifluoromethyl,
or represent phenyl or phenyl-C₁-C₂-alkyl, in each case optionally substituted once or twice in an identical or different manner by fluorine, chlorine, methyl, ethyl, methoxy, ethoxy, methylthio, trifluoromethyl, trifluoromethoxy, trifluoromethylthio, dimethylamino, diethylamino, cyano, nitro, -COOR¹⁵ or -CONR¹⁶R¹⁷,
or represent -OR⁶ or -NR⁵R⁶,
R⁹ and R¹⁰ independently of one another represent methyl, ethyl or n- or isopropyl;
R¹¹ represents -OR⁶, -NR⁵R⁶ or -N(R⁵)-COOR⁶,
and
R¹², R¹³ and R¹⁴ independently of one another represent methyl, ethyl or n- or isopropyl.
R¹⁵ represents hydrogen, methyl, ethyl, or n- or isopropyl; or C₁-C₂-halogenoalkyl having 1 to 5 identical or different halogen atoms, such as F and Cl atoms; or cyclopropyl, cyclopentyl or cyclohexyl, in each case optionally substituted once to three times in an identical or different manner by fluorine, chlorine, methyl, ethyl, n- or isopropyl or C₁-C₂-halogenoalkyl having 1 to 5 identical or different halogen atoms, such as F and Cl atoms,
or represents phenyl which is optionally substituted once or twice in an identical or different manner by fluorine, chlorine, methyl, ethyl, methoxy, ethoxy, methylthio, trifluoromethyl, trifluoromethoxy, trifluoromethylthio, dimethylamino, diethylamino, cyano, nitro, C₁-C₄-alkoxycarbonyl or di(C₁-C₄)alkylaminocarbonyl
R¹⁶ and R¹⁷ independently of one another represent hydrogen, C₁-C₄-alkyl, methoxy, ethoxy or C₁-C₃-halogenoalkyl having 1 to 5 identical or different halogen atoms, such as F and Cl atoms; or allyl which is optionally substituted once or several times by fluorine and/or chlorine; or cyclopropyl, cyclopentyl, cyclohexyl, cyclopropylmethyl, cyclopentylmethyl or cyclohexylmethyl, in each case optionally substituted once to three times in an identical or different manner by fluorine, chlorine, methyl, ethyl, n- or isopropyl or trifluoromethyl,
or represent phenyl or phenyl-C₁-C₂-alkyl, in each case optionally substituted once or twice in an identical or different manner by fluorine, chlorine, methyl, ethyl, methoxy, ethoxy, methylthio, trifluoromethyl, trifluoromethoxy, trifluoromethylthio, dimethylamino, diethylamino, cyano, nitro, C₁-C₄-alkoxycarbonyl or di(C₁-C₄)alkylaminocarbonyl,
or represent -OR⁶ or -NR⁵R⁶.

3. Compounds of the formula (X) 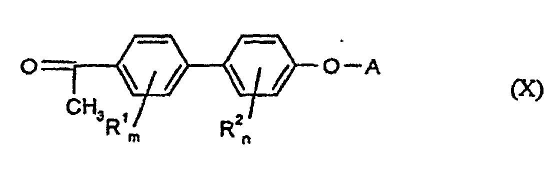 in which
A, R¹, R², m and n have the meaning given in Claim 1.

4. Compounds of the formula (XI) 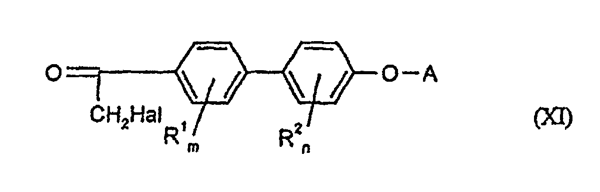 in which
A, R¹, R², m and n have the meaning given in Claim 1 and
Hal represents chlorine or bromine.

5. Compounds of the formula (XII) 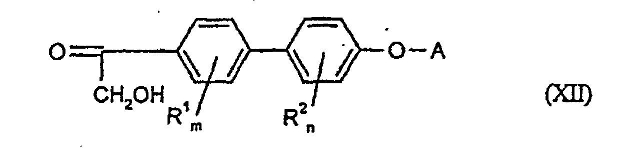 in which
A, R¹, R², m and n have the meaning given in Claim 1.

6. Compounds of the formula (XIII) 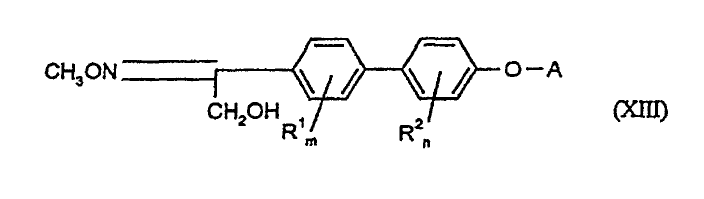 in which
A, R¹, R², m and n have the meaning given in Claim 1.

7. Compounds of the formula (XIV) 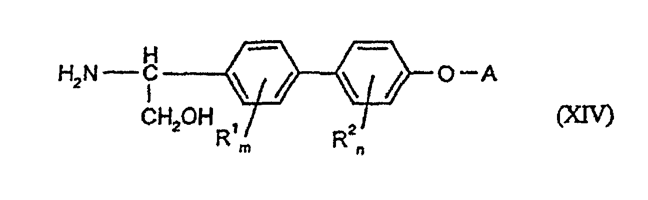 in which
A, R¹, R², m and n have the meaning given in Claim 1.

8. Compounds of the formula (XVI) 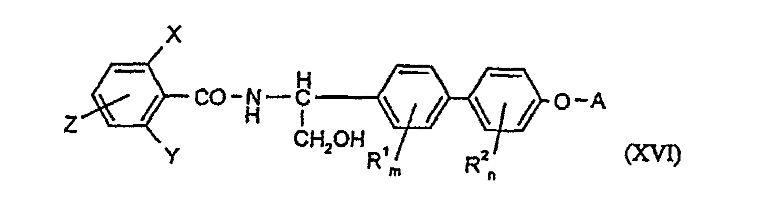 in which
A, R¹, R², m, n, X, Y and Z have the meaning given in Claim 1.

9. Compounds of the formula (XVII) 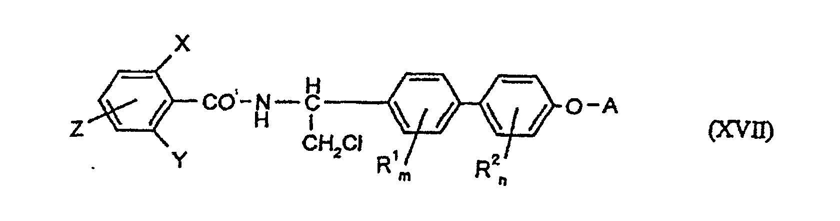 in which
A, R¹, R², m, n, X, Y and Z have the meaning given in Claim 1.

10. Process for the preparation of compounds of the formula (I) according to Claim 1, **characterized in that** hydroxybiphenyloxazolines of the formula (II) 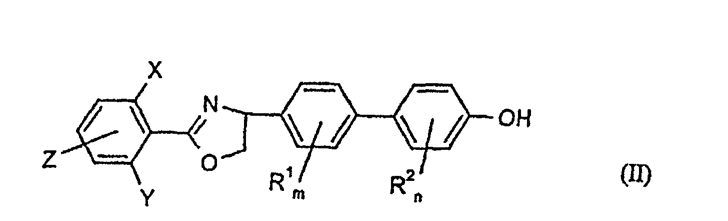 in which
X, Y, Z, R¹, R², m and n have the meaning given in Claim 1,
are reacted with a compound of the formula (III)
M-A (III)
in which
A has the meaning given in Claim 1 and
M represents a leaving group,
if appropriate in the presence of a base and/or a catalyst and if appropriate in the presence of a diluent.

11. Pest control composition, **characterized by** a content of at least one compound of the formula (I) according to Claim 1.

12. Use of compounds of the formula (I) according to Claim 1 for controlling pests.

13. Method of controlling pests, **characterized in that** compounds of the formula (I) according to Claim 1 are allowed to act on pests and/or their environment.

14. Process for the preparation of pest control compositions, **characterized in that** compounds of the formula (I) according to Claim 1 are mixed with extenders and/or surface-active agents.

15. Use of compounds of the formula (I) according to Claim 1 for the preparation of pest control compositions.

## Revendications

1. Composés de formule (I) 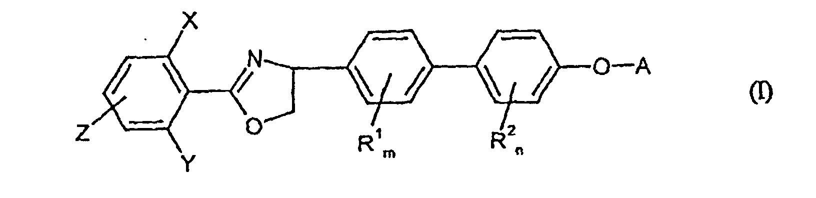 dans laquelle
X représente l'hydrogène, le fluor ou le chlore,
Y représente le fluor, le chlore ou le groupe méthyle,
Z représente l'hydrogène, le fluor, le chlore, un groupe alkyle en C₁ à C₄, alkoxy en C₁ à C₄ ou di(alkyle en C₁ à C₄)amino,
R¹ et R² représentent, indépendamment l'un de l'autre, le fluor, le chlore, un groupe alkyle en C₁ à C₄, alkoxy en C₁ à C₄, alkylthio en C₁ à C₄, halogénalkyle en C₁ à C₄, halogénalkoxy en C₁ à C₄ ou halogénalkylthio en C₁ à C₄,
m et n ont, indépendamment l'un de l'autre, la valeur 0, 1 ou 2,
A représente le groupement (CH₂)ₚ-(CR³R⁴)_{q}-(CH₂)ᵣ-R, où
R³ et R⁴ représentent, indépendamment l'un de l'autre, l'hydrogène ou un groupe alkyle en C₁ à C₄,
p, q et r ont, indépendamment les uns des autres, la valeur 0, 1, 2 ou 3, l'un au moins des indices étant différent de 0 et la somme des indices n'étant pas supérieure à 5,
R représente un groupe cyano ; l'un des hétérocycles suivants 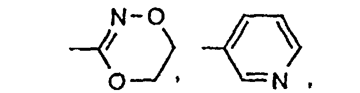 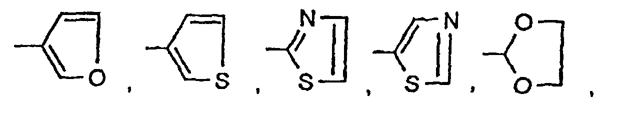 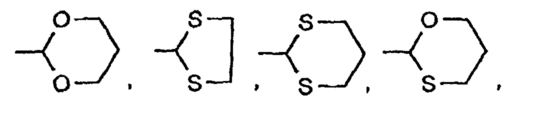 qui portent éventuellement un à trois substituants, identiques ou différents, fluoro, chloro, bromo, méthyle, éthyle, n-propyle, isopropyle ou trifluorométhyle ;
ou l'un des groupements suivants :
(a) ―CO-R⁵
(b) ―CO-OR⁶
(c) ―CO-NR⁷R⁸
(d) ―CS-NR⁷R⁸
(e) 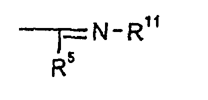
(f) 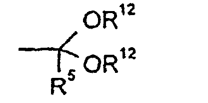
(g) 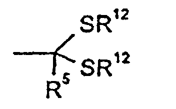
(h) 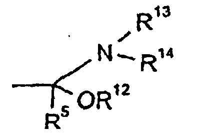
(i) 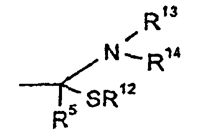
(j) 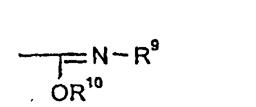
(k) 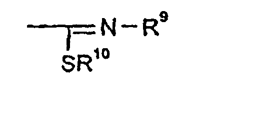
dans lesquels
R⁵ représente l'hydrogène ; un groupe méthyle, éthyle, n-propyle, isopropyle, les groupes butyle isomères, les groupes pentyle isomères, les groupes hexyle isomères ; un groupe halogénalkyle en C₁ ou C₂ ayant 1 à 5 atomes d'halogènes identiques ou différents tels que des atomes de F- et Cl-, un groupe alcényle en C₃ à C₆, un groupe halogénalkyle en C₃ à C₆ ayant 1 à 5 atomes d'halogènes identiques ou différents tels que des atomes de F- et Cl- ; un groupe cyclopropyle, cyclopentyle ou cyclohexyle dont chacun est éventuellement substitué une à trois fois identiques ou différentes par du fluor, du chlore, un radical méthyle, éthyle, n-propyle, isopropyle ou halogénalkyle en C₁ ou C₂ ayant 1 à 5 atomes d'halogènes identiques ou différents tels que des atomes de F- et Cl-,
ou bien un groupe phényle portant éventuellement un ou deux substituants, identiques ou différents, fluor, chlore, méthyle, éthyle, méthoxy, éthoxy, méthylthio, trifluorométhyle, trifluorométhoxy, trifluorométhylthio, diméthylamino, diéthylamino, méthylcarbonylamino, éthylcarbonylamino, méthylcarbonyl-méthylamino, cyano, nitro, -COOR¹⁵ ou -CONR¹⁶R¹⁷,
R⁶ représente l'hydrogène, un groupe méthyle, éthyle, n-propyle, isopropyle, n-butyle, isobutyle, sec.-butyle ou tertio-butyle ; un groupe halogénalkyle en C₁ ou C₂ ayant 1 à 5 atomes d'halogènes identiques ou différents tels que des atomes de F- et Cl- ; un groupe allyle portant éventuellement un ou plusieurs substituants fluoro et/ou chloro ; un groupe cyclopropyle, cyclopentyle, cyclohexyle, cyclopropyl(alkyle en C₁ ou C₂), cyclopentyl(alkyle en C₁ ou C₂) ou cyclohexyl(alkyle en C₁ ou C₂) dont chacun porte éventuellement un à trois substituants, identiques ou différents, fluoro, chloro, méthyle, éthyle, n-propyle, isopropyle ou halogénalkyle en C₁ ou C₂ ayant 1 à 5 atomes d'halogènes identiques ou différents tels que des atomes de F- et de Cl-,
ou bien un groupe phényl-(alkyle en C₁ ou C₂) ou un groupe naphtyl-(alkyle en C₁ ou C₂) portant chacun, le cas échéant, un ou deux substituants, identiques ou différents, fluoro, chloro, méthyle, éthyle, méthoxy, éthoxy, méthylthio, trifluorométhyle, trifluorométhoxy, trifluorométhylthio, diméthylamino, diéthylamino, cyano, nitro, -COOR¹⁵ ou -CONR¹⁶R¹⁷,
R⁷ et R⁸ représentent, indépendamment l'un de l'autre, l'hydrogène, un groupe alkyle en C₁ à C₄, méthoxy, éthoxy, halogénalkyle en C₁ à C₃ ayant 1 à 5 atomes d'halogènes identiques ou différents tels que des atomes de F- et de Cl- ; un groupe allyle portant éventuellement un ou plusieurs substituants fluoro et/ou chloro ; un groupe cyclopropyle, cyclopentyle, cyclohexyle, cyclopropylméthyle, cyclopentylméthyle ou cyclohexylméthyle portant chacun, le cas échéant, un à trois substituants, identiques ou différents, fluoro, chloro, méthyle, éthyle, n-propyle, isopropyle ou trifluorométhyle,
ou bien un groupe phényle ou phényl(alkyle en C₁ ou C₂) portant chacun, le cas échéant, un ou deux substituants, identiques ou différents, fluoro, chloro, méthyle, éthyle, méthoxy, éthoxy, méthylthio, trifluorométhyle, trifluorométhoxy, trifluorométhylthio, diméthylamino, diéthylamino, cyano, nitro, -COOR¹⁵ ou -CONR¹⁶R¹⁷,
ou un groupe -OR⁶ ou -NR⁵R⁶,
R⁹ et R¹⁰ représentent, indépendamment l'un de l'autre, un groupe méthyle, éthyle, n-propyle ou isopropyle ;
R¹¹ est un groupe -OR⁶, -NR⁵R⁶ ou -N(R⁵)-COOR⁶,
et
R¹², R¹³ et R¹⁴ représentent, indépendamment l'un de l'autre, un groupe méthyle, éthyle, n-propyle ou isopropyle,
R¹⁵ représente l'hydrogène, un groupe méthyle, éthyle, n-propyle, isopropyle ; un groupe halogénalkyle en C₁ ou C₂ ayant 1 à 5 atomes d'halogènes identiques ou différents tels que des atomes de F- et de Cl- ; un groupe cyclopropyle, cyclopentyle ou cyclohexyle portant chacun, le cas échéant, un à trois substituants, identiques ou différents, fluoro, chloro, méthyle, éthyle, n-propyle, isopropyle ou halogénalkyle en C₁ ou C₂ ayant 1 à 5 atomes d'halogènes identiques ou différents tels que des atomes de F- et de Cl-,
ou un groupe phényle portant, le cas échéant, un ou deux substituants, identiques ou différents, fluoro, chloro, méthyle, éthyle, méthoxy, éthoxy, méthylthio, trifluorométhyle, trifluorométhoxy, trifluorométhylthio, diméthylamino, diéthylamino, cyano, nitro, (alkoxy en C₁ à C₄)carbonyle ou di(alkyle en C₁ à C₄)aminocarbonyle,
R¹⁶ et R¹⁷ représentent, indépendamment l'un de l'autre, l'hydrogène, un groupe alkyle en C₁ à C₄, méthoxy, éthoxy, halogénalkyle en C₁ à C₃ ayant 1 à 5 atomes d'halogènes identiques ou différents tels que des atomes de F- et de Cl- ; un groupe allyle éventuellement substitué une ou plusieurs fois par du fluor et/ou du chlore ; un groupe cyclopropyle, cyclopentyle, cyclohexyle, cyclopropylméthyle, cyclopentylméthyle ou cyclohexylméthyle portant chacun, le cas échéant, un à trois substituants, identiques ou différents, fluoro, chloro, méthyle, éthyle, n-propyle, isopropyle ou trifluorométhyle,
ou bien un groupe phényle ou un groupe phényl-(alkyle en C₁ ou C₂) portant chacun, le cas échéant, un ou deux substituants, identiques ou différents, fluoro, chloro, méthyle, éthyle, méthoxy, éthoxy, méthylthio, trifluorométhyle, trifluorométhoxy, trifluorométhylthio, diméthylamino, diéthylamino, cyano, nitro, (alkoxy en C₁ à C₄) - carbonyle ou di(alkyle en C₁ à C₄) aminocarbonyle,
ou bien un groupe -OR⁶ ou -NR⁵R⁶.

2. Composés de formule (I) suivant la revendication 1, dans laquelle
X représente l'hydrogène, le fluor ou le chlore,
Y représente le fluor, le chlore ou un groupe méthyle,
Z représente l'hydrogène, le fluor, le chlore, un groupe alkyle en C₁ à C₄, alkoxy en C₁ à C₄ ou di(alkyle en C₁ à C₄)amino,
R¹ et R² représentent, indépendamment l'un de l'autre, le fluor, le chlore, un groupe alkyle en C₁ à C₄, alkoxy en C₁ à C₄, alkylthio en C₁ à C₄, halogénalkyle en C₁ à C₄, halogénalkoxy en C₁ à C₄ ou halogénalkylthio en C₁ à C₄,
m et n ont, indépendamment l'un de l'autre, la valeur 0, 1 ou 2,
A représente le groupement (CH₂)ₚ-(CR³R⁴)_{q}-(CH₂)ᵣ-R,
où
R³ et R⁴ xreprésentent, indépendamment l'un de l'autre, l'hydrogène ou un groupe alkyle en C₁ à C₄,
p, q et r ont, indépendamment les uns des autres, la valeur 0, 1, 2 ou 3, l'un au moins des indices étant différent de 0 et la somme des indices n'étant pas supérieure à 5,
R représente un groupe cyano ; l'un des hétérocycles suivants 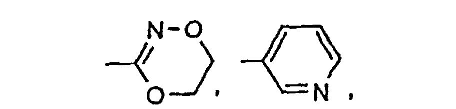 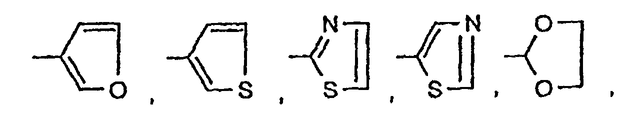 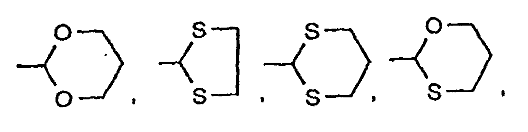 qui portent éventuellement un à trois substituants, identiques ou différents, fluoro, chloro, bromo, méthyle, éthyle, n-propyle, isopropyle ou trifluorométhyle ;
ou bien l'un des groupements suivants :
(a) ―CO-R⁵
(b) ―CO-OR⁶
(c) ―CO-NR⁷R⁸
(d) ―CS-NR⁷R⁸
(e) 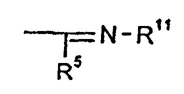
(f) 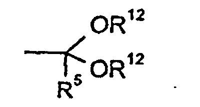
(g) 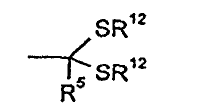
(h) 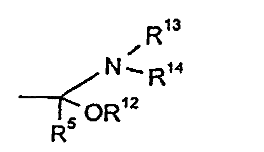
(i) 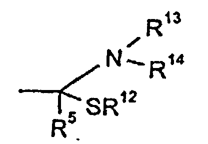
(j) 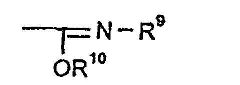
(k) 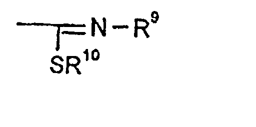
dans lesquels
R⁵ représente l'hydrogène ; un groupe méthyle, éthyle, n-propyle, isopropyle, les groupes butyle isomères, les groupes pentyle isomères, les groupes hexyle isomères ; un groupe halogénalkyle en C₁ ou C₂ ayant 1 à 5 atomes d'halogènes identiques ou différents tels que des atomes de F- et de Cl-, un groupe alcényle en C₃ à C₆, halogénalkyle en C₃ à C₆ ayant 1 à 5 atomes d'halogènes identiques ou différents tels que des atomes de F- et Cl- ; un groupe cyclopropyle, cyclopentyle ou cyclohexyle portant éventuellement dans chaque cas un à trois substituants, identiques ou différents, fluoro, chloro, méthyle, éthyle, n-propyle, isopropyle ou halogénalkyle en C₁ ou C₂ ayant 1 à 5 atomes d'halogènes identiques ou différents tels que des atomes de F- et Cl-,
ou bien un groupe phényle éventuellement substitué une ou deux fois identiques ou différntes, par un radical par un radical fluoro, chloro, méthyle, éthyle, méthoxy, éthoxy, méthylthio, trifluorométhyle, trifluorométhoxy, trifluorométhylthio, diméthylamino, diéthylamino, méthylcarbonylamino, éthylcarbonylamino, méthylcarbonyl-méthylamino, cyano, nitro, -COOR¹⁵ ou -CONR¹⁶R¹⁷,
R⁶ représente l'hydrogène, un groupe méthyle, éthyle, n-propyle, isopropyle, n-butyle, isobutyle, sec.-butyle ou tertio-butyle ; un groupe halogénalkyle en C₁ ou C₂ ayant 1 à 5 atomes d'halogènes identiques ou différents tels que des atomes de F- et Cl- ; un groupe allyle éventuellement substitué une ou plusieurs fois par du fluor et/ou du chlore ; un groupe cyclopropyle, cyclopentyle, cyclohexyle, cyclopropyl-(alkyle en C₁ ou C₂), cyclopentyl-(alkyle en C₁ ou C₂) ou cyclohexyl-(alkyle en C₁ ou C₂) portant chacun, le cas échéant, un à trois substituants, identiques ou différents, fluoro, chloro, méthyle, éthyle, n-propyle, isopropyle ou halogénalkyle en C₁ ou C₂ ayant 1 à 5 atomes d'halogènes identiques ou différents tels que des atomes de F- et Cl-,
ou bien un groupe phényl-(alkyle en C₁ ou C₂) ou un groupe naphtyl-(alkyle en C₁ ou C₂) dont chacun porte, le cas échéant, un ou deux substituants, identiques ou différents, fluoro, chloro, méthyle, éthyle, méthoxy, éthoxy, méthylthio, trifluorométhyle, trifluorométhoxy, trifluorométhylthio, diméthylamino, diéthylamino, cyano, nitro, -COOR¹⁵ ou -CONR¹⁶R¹⁷,
R⁷ et R⁸ représentent, indépendamment l'un de l'autre, l'hydrogène, un groupe alkyle en C₁ à C₄, méthoxy, éthoxy, halogénalkyle en C₁ à C₃ ayant 1 à 5 atomes d'halogènes identiques ou différents tels que des atomes de F- et Cl- ; un groupe allyle éventuellement substitué une ou plusieurs fois par du fluor et/ou chlore ; un groupe cyclopropyle, cyclopentyle, cyclohexyle, cyclopropylméthyle, cyclopentylméthyle ou cyclohexylméthyle portant chacun, le cas échéant, un à trois substituants, identiques ou différents, fluoro, chloro, méthyle, éthyle, n-propyle, isopropyle ou trifluorométhyle,
ou bien un groupe phényle ou phényl(alkyle en C₁ ou C₂) portant chacun, le cas échéant, un ou deux substituants, identiques ou différents, fluoro, chloro, méthyle, éthyle, méthoxy, éthoxy, méthylthio, trifluorométhyle, trifluorométhoxy, trifluorométhylthio, diméthylamino, diéthylamino, cyano, nitro, -COOR¹⁵ ou -CONR¹⁶R¹⁷,
ou un groupe -OR⁶ ou -NR⁵R⁶,
R⁹ et R¹⁰ représentent, indépendamment l'un de l'autre, un groupe méthyle, éthyle, n-propyle ou isopropyle ;
R¹¹ est un groupe -OR⁶, -NR⁵R⁶ ou -N(R⁵)-COOR⁶,
et
R¹², R¹³ et R¹⁴ représentent, indépendamment l'un de l'autre, un groupe méthyle, éthyle, n-propyle ou isopropyle,
R¹⁵ représente l'hydrogène, un groupe méthyle, éthyle, n-propyle, isopropyle ; un groupe halogénalkyle en C₁ ou C₂ ayant 1 à 5 atomes d'halogènes identiques ou différents tels que des atomes de F- et de Cl- ; un groupe cyclopropyle, cyclopentyle ou cyclohexyle portant chacun, le cas échéant, un à trois substituants, identiques ou différents, fluoro, chloro, méthyle, éthyle, n-propyle, isopropyle ou halogénalkyle en C₁ ou C₂ ayant 1 à 5 atomes d'halogènes identiques ou différents tels que des atomes de F- et de Cl-,
ou bien un groupe phényle portant éventuellement un ou deux substituants, identiques ou différents, fluoro, chloro, méthyle, éthyle, méthoxy, éthoxy, méthylthio, trifluorométhyle, trifluorométhoxy, trifluorométhylthio, diméthylamino, diéthylamino, cyano, nitro, (alkoxy en C₁ à C₄)carbonyle ou di(alkyle en C₁ à C₄)aminocarbonyle,
R¹⁶ et R¹⁷ représentent, indépendamment l'un de l'autre, l'hydrogène, un groupe alkyle en C₁ à C₄, méthoxy, éthoxy, halogénalkyle en C₁ à C₃ ayant 1 à 5 atomes d'halogènes identiques ou différents tels que des atomes de F- et de Cl- ; un groupe allyle éventuellement substitué une ou plusieurs fois par du fluor et/ou du chlore ; un groupe cyclopropyle, cyclopentyle, cyclohexyle, cyclopropylméthyle, cyclopentylméthyle ou cyclohexylméthyle portant chacun, le cas échéant, un à trois substituants, identiques ou différents, fluoro, chloro, méthyle, éthyle, n-propyle, isopropyle ou trifluorométhyle,
ou bien un groupe phényle ou un groupe phényl-(alkyle en C₁ ou C₂) portant chacun, le cas échéant, un ou deux substituants, identiques ou différents, fluoro, chloro, méthyle, éthyle, méthoxy, éthoxy, méthylthio, trifluorométhyle, trifluorométhoxy, trifluorométhylthio, diméthylamino, diéthylamino, cyano, nitro, (alkoxy en C₁ à C₄)-carbonyle ou di(alkyle en C₁ à C₄)aminocarbonyle,
ou bien un groupe -OR⁶ ou -NR⁵R⁶.

3. Composés de formule (X) 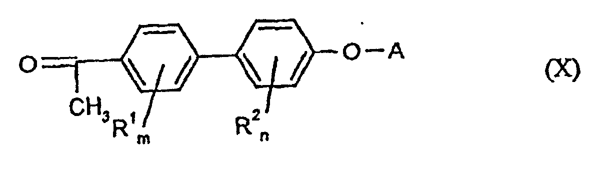 dans laquelle
A, R¹, R², m et n ont la définition indiquée dans la revendication 1.

4. Composés de formule (XI) 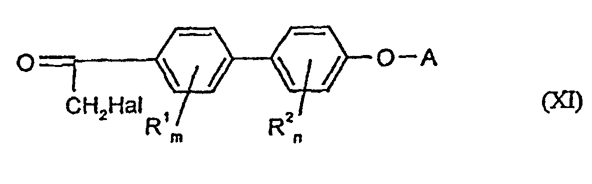 dans laquelle
A, R¹, R², m et n ont la définition indiquée dans la revendication 1 et
Hal représente le chlore ou le brome.

5. Composés de formule (XII) 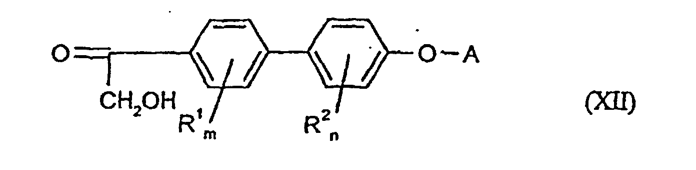 dans laquelle
A, R¹, R², m et n ont la définition indiquée dans la revendication 1.

6. Composés de formule (XIII) 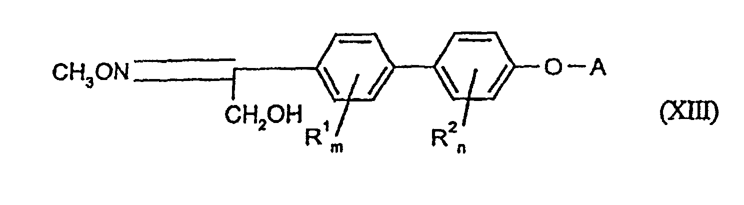 dans laquelle
A, R¹, R², m et n ont la définition indiquée dans la revendication 1.

7. Composés de formule (XIV) 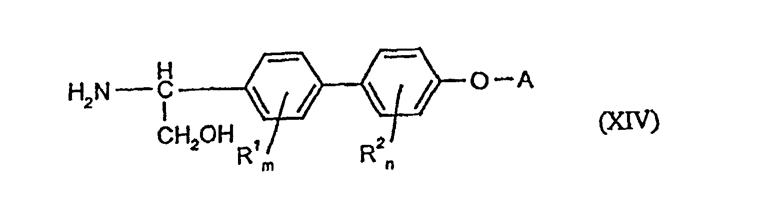 dans laquelle
A, R¹, R², m et n ont la définition indiquée dans la revendication 1.

8. Composés de formule (XVI) 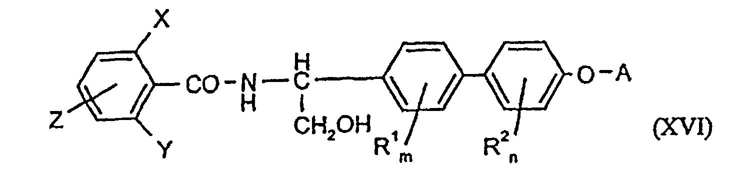 dans laquelle
A, R¹, R², m, n, X, Y et Z ont la définition indiquée dans la revendication 1.

9. Composés de formule (XVII) 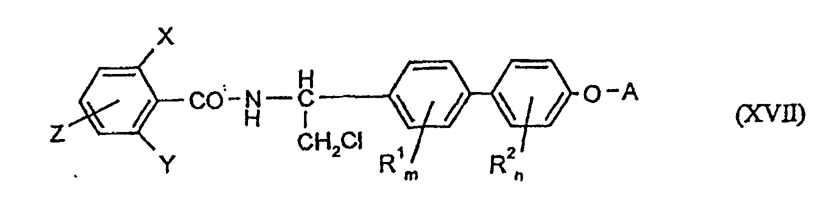 dans laquelle
A, R¹, R², m, n, X, Y et Z ont la définition indiquée dans la revendication 1.

10. Procédé de production de composés de formule (I) suivant la revendication 1, **caractérisé en ce qu'**on fait réagir des hydroxybiphényloxazolines de formule (II) 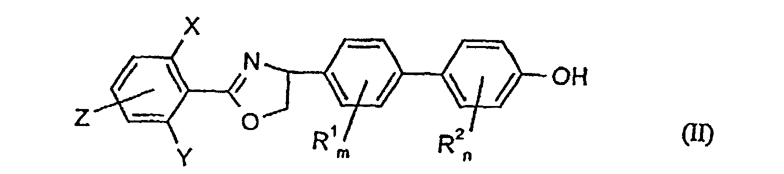 dans laquelle
X, Y, Z, R¹, R², m et n ont la définition indiquée dans la revendication 1,
avec un composé de formule (III)
M-A (III)
dans laquelle
A a la définition dans la revendication 1 et
M représente un groupe partant,
en présence éventuelle d'une base et/ou d'un catalyseur et, le cas échéant en présence d'un diluant.

11. Compositions pesticides, **caractérisées par** une teneur en au moins un composé de formule (I) suivant la revendication 1.

12. Utilisation de composés de formule (I) suivant la revendication 1 pour combattre des parasites.

13. Procédé pour combattre des parasites, **caractérisé en ce qu'**on fait agir des composés de formule (I) suivant la revendication 1 sur les parasites et/ou sur leur milieu.

14. Procédé de préparation de compositions pesticides, **caractérisé en ce qu'**on mélange des composés de formule (I) suivant la revendication 1 avec des diluants et/ou des agents tensio-actifs.

15. Utilisation de composés de formule (I) suivant la revendication 1 pour la préparation de compositions pesticides.
